# EUROPÄISCHE PATENTSCHRIFT

(11) **EP 0 619 374 B1**
(45) Veröffentlichungstag und Bekanntmachung des Hinweises auf die Patenterteilung: **13.08.1997**
(21) Anmeldenummer: 94105140.1
(22) Anmeldetag: 31.03.1994
(51) Int. Cl.: C07C 255/24, C07F 9/6561, C12Q 1/32, C07C 211/52

(54) **Verfahren zur kolorimetrischen Bestimmung eines Analyten mittels Benzylalkoholdehydrogenase und einem chromogenen Redoxindikator**
Colorimetric determination of an analyte using benzyl alcohol dehydrogenase and a chromogenic redox-indicator
Détermination colorimétrique d'analyte utilisant une déhydrogénèse d'alcool benzylique et un indicateur redox chromogénique

(30) Priorität: 08.04.1993 DE 4311460
(43) Veröffentlichungstag der Anmeldung: 12.10.1994
(73) Patentinhaber: BOEHRINGER MANNHEIM GMBH, 68298 Mannheim (DE)
(72) Erfinder: Hoenes, Joachim, D-64673 Zwingenberg (DE); Unkrig, Volker, D-68526 Ladenburg (DE)

(56) Entgegenhaltungen:
- EP-A- 0 433 854
- EP-A- 0 441 222
- AGRICULTURAL AND BIOLOGICAL CHEMISTRY, Bd. 48, Nr. 5, Mai 1984 TOKYO, Seiten 1161-1171, YAMANAKA ET AL. 'Identification and characterization of nicotinamide adenine ....'
- ANALYTICAL BIOCHEMISTRY, Bd. 137, Nr. 1, 15.Februar 1984 NEW YORK NY, Seiten 74-79, KOVAR ET AL. 'A new photometric assay for blood alcohol'
- JAMES 'The theory of photographic process. 3rd ed., chapter17 "Principles and chemistry of color photography" Seite 382 - 396' 1966 , MC MILLAN , NEW YORK NY * Formel 13, Seite 384 *
- THE JOURNAL OF ORGANIC CHEMISTRY, Bd. 32, Nr. 1, Januar 1967 WASHINGTON DC, Seiten 158-162, HAYS ET AL. 'p-Nitrosophenol chemistry. ....'
- AGRICULTURAL AND BIOLOGICAL CHEMISTRY, Bd. 48, Nr. 5, Mai 1984 Tokyo, JP, Seiten 1161-1171, K. YAMANAKA et al: "Identification and characterization of nicotinamide adenine dinucleotide-dependent p-hydroxybenzyl alcohol dehydrogenase from Rhodopseudomonas acidophila M402"
- ANALYTICAL BIOCHEMISTRY, Bd. 137, Nr. 1, 15. Februar 1984, New York, US, Seiten 74-79, J. KOVAR et al: "A new photometric assay for blood alcohol"
- T.H. JAMES, The theory of photographic process, 3rd ed., chapter 17, "Principles and chemistry of color photography", Seite 382 - 396, 1966, MC MILLAN, New York, US

## Beschreibung

Die Erfindung betrifft ein Verfahren zur kolorimetrischen Bestimmung von NAD(P)H oder eines Analyten, der ein Substrat eines unter Bildung von NAD(P)H reagierenden Enzyms ist, mit einem NAD(P)H-oxidierenden Enzym und einem chromogenen Redox-Indikator. Weiter betrifft die Erfindung ein Mittel zur Bestimmung von NAD(P)H oder eines Analyten, der ein Substrat eines unter Bildung von NAD(P)H reagierenden Enzyms ist, enthaltend Benzylalkoholdehydrogenase und chromogene Nitrosoanilinverbindungen. Weiter betrifft die Erfindung neue Nitrosoanilinverbindungen, deren Herstellung sowie deren Verwendung zur kolorimetrischen enzymatischen Bestimmung eines Analyten.

Enzymatische Oxidationen ermöglichen in der Analytik den Nachweis und die Bestimmung von Substanzen in verschiedensten Probenmaterialien. Hierbei wirkt ein oxidierendes Enzym in Gegenwart eines die Elektronen der Oxidationsreaktion aufnehmenden Akzeptors auf ein entsprechendes Enzymsubstrat ein. Die Reduktion des Elektronenakzeptors zeigt die Anwesenheit des Enzymsubstrates an. Hierbei hat es sich bisher als besonders vorteilhaft erwiesen, wenn der reduzierte Elektronenakzeptor durch Farbbildung nachgewiesen werden kann, da dies nicht notwendigerweise nur mittels teurer Meßapparaturen möglich ist, sondern gegebenenfalls auch visuell erfolgen kann.

Zur kolorimetrischen Bestimmung von Analyten werden häufig Reaktionen eingesetzt, bei denen eine NAD(P)-abhängige Dehydrogenase beteiligt ist. Oft ist der Analyt ein Substrat dieser Dehydrogenase selbst und wird unter NAD(P)H-Bildung oxidiert, oder eine Reaktionskaskade verschiedener Vorreaktionen wandelt den Analyten in ein Substrat einer NAD(P)-abhängigen Dehydrogenase um, das dann von dieser unter NAD(P)H-Bildung oxidiert wird. Das entstandene NAD(P)H kann beispielsweise UV-spektrometrisch bestimmt werden. Diese Methode ist recht unempfindlich und erfordert teure Meßapparaturen. Eine visuelle Auswertung ist nicht möglich.

Aus beispielsweise DE-OS-2147 466 ist der kolorimetrische Nachweis von NAD(P)H in Gegenwart des Enzym Diaphorase als Redox-Katalysator mit Tetrazoliumsalzen als direkten Elektronenakzeptoren bekannt. Dort wird beschrieben, daß Lactat unter Katalyse von Lactatdehydrogenase mit Nikotinamid-Adenin-Dinukleotid zu Pyruvat und reduziertem Nikotinamid-Adenin-Dinukleotid umgesetzt wird. Das gebildete NADH reagiert dann in Gegenwart des Enzyms Diaphorase mit Tetrazoliumsalzen unter Bildung von NAD+ und farbigen Formazanen, deren Konzentration photometrisch oder visuell bestimmt werden kann. Nachteile dieses Verfahrens liegen in der Unspezifität der Diaphorase und der Instabilität, insbesondere der Licht- und Basenempfindlichkeit der Tetrazoliumsalze (siehe auch H. U. Bergmeyer, "Grundlagen der enzymatischen Analyse", 3. Auflage, 1977, Seite 90-95, Verlag Chemie, Weinheim).

Zum kolorimetrischen Nachweis von NAD(P)H werden in der EP-A-0 431 456 statt Tetrazoliumsalzen aromatische Nitrosoverbindungen als Elektronenakzeptoren beschrieben. NAD(P)H wird wiederum in Gegenwart des Enzyms Diaphorase als Reduktionskatalysator mit aromatischen Nitrosoverbindungen oxidiert. Die aromatischen Nitrosoverbindungen werden dabei zu elektronenreichen aromatischen Aminen reduziert. Da diese Amine an sich überhaupt nicht oder nur sehr schwach gefärbt sind, müssen sie für eine kolorimetrische Bestimmung entweder mit einer gefällten Heteropolysäure oder mit einem farberzeugenden Kupplungsreagenz in Kombination mit einem Oxidationsmittel zu einem farbigen Reaktionsprodukt umgesetzt werden. Die Anzahl der verschiedenen Reagenzien und Reaktionsstufen machen solche Teste kompliziert und teuer. Die grau-blauen Farben der reduzierten Heteropolysäuren sind insbesondere für eine visuelle Auswertung wenig geeignet. Die oxidative Kupplung mit einem farberzeugenden Kupplungsreagenz in Gegenwart eines Oxidationsmittels hat den Nachteil, daß die Reduktion des Nitrosoaromaten zum Amin getrennt von der anschließenden oxidativen Kupplung des Amins mit dem farberzeugenden Kupplungsreagenz durchgeführt werden muß, da das dafür benötigte Oxidationsmittel die vorhergehende Reduktionsreaktion stören würde.

Ferner werden bei der Reduktion eines Äquivalents der aromatischen Nitrosoverbindung zum Amin zwei Äquivalente eines Analyten beziehungsweise NAD(P)H reduziert, was eine Einschränkung der Empfindlichkeit des Nachweisverfahrens, insbesondere bei geringen Analytkonzentrationen bedeutet.

Aufgabe der vorliegenden Erfindung war es daher, die Nachteile des Standes der Technik zu beseitigen und ein pfindlicheres, weniger störanfälliges, einfacher durchführbares kolorimetrisches Nachweisverfahren für NAD(P)H, insbesondere für Analyten, die mit einer NAD(P)H-abhängigen Dehydrogenase bestimmt werden können, zur Verfügung zu stellen, das über den gesamten sichtbaren Wellenlängenbereich visuell gut auswertbare Farben als Folge einer spezifischen Reaktion liefert. Insbesondere sollte in dem Verfahren der Analyt beziehungsweise NAD(P)H direkt in einer Stufe mit einem chromogenen Redox-Indikator nachweisbar sein, ohne daß weitere Folgereaktionen nötig wären. Die Aufgabe wird gelöst durch die Erfindung, wie sie in den Ansprüchen charakterisiert ist.

Überraschenderweise wurde gefunden, daß NAD(P)H sehr vorteilhaft mit dem NAD(P)H-abhängigen Enzym Benzylalkoholdehydrogenase und einer Nitrosoanilinverbindung als direktem Gegenstand der Erfindung ist ein Verfahren zur kolorimetrischen Bestimmung von NAD(P)H oder eines Analyten, der ein Substrat eines unter Bildung von NAD(P)H reagierenden Enzyms ist, mittels Oxidation mit einem NAD(P)H-oxidierenden Enzym in Anwesenheit eines direkten chromogenen Elektronenakzeptors und Bestimmung des reduzierten Elektronenakzeptors als Maß für die Menge an NAD(P)H, dadurch gekennzeichnet, daß NAD(P)H mit dem Enzym Benzylalkoholdehydrogenase in Anwesenheit einer Nitrosoanilinverbindung der allgemeinen Formel I 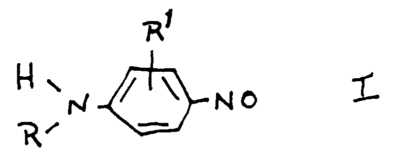 wobei
- R1 =: Wasserstoff, Hydroxy, Halogen, C₁-C₆ Alkoxy oder Alkylthio, C₆-C₁₀ Aryloxy oder Arylthio, C₁-C₆ Alkyl oder C₂-C₅ Alkylen, gegebenenfalls substituiert durch Carboxy, P0₃H₂, Di-( C₁-C₆) alkylphosphinyl oder SO₃H, Amino, gegebenenfalls ein- oder zweimal substituiert durch C₁-C₆ Alkyl, das gegebenenfalls wiederum durch Hydroxy oder Carboxy, P0₃H₂, Dialkylphosphinyl oder SO₃H substituiert sein kann und
- R =: einen bei Elektronen-Konjugation mit einem Chinondiimin-System farberzeugenden, Rest darstellt,
als Elektronenakzeptor umgesetzt wird, dabei die Nitrosoanilinverbindung zu einem farbigen Chinondiimin enzymatisch reduziert wird und die Farbe des Chinondiimins als Maß für die Menge an NAD(P)H gemessen wird.

Bevorzugter Rest R1 ist Wasserstoff.

Erfindungsgemäß wird unter "Analyt" eine solche Substanz verstanden, die enzymatisch durch eine NAD-abhängige Dehydrogenase oxidiert wird. In vielen Fällen wird der Analyt diejenige Substanz sein, die in der zu untersuchenden Probe direkt nachgewiesen werden soll. Beispielsweise kann Lactat direkt mit Lactatdehydrogenase und NAD oxidiert und über den Nachweis des entstehenden NADH kolorimetrisch bestimmt werden. Es ist jedoch auch möglich, daß der Analyt durch eine oder mehrere vorgeschaltete Reaktionen aus einer anderen Substanz gebildet wird, so daß durch kolorimetrische Bestimmung des Analyten beziehungsweise von NAD(P)H auf die Konzentration der Ausgangssubstanz geschlossen werden kann. Der Analyt ist in der vorliegenden Erfindung diejenige Substanz, die als Substrat einer NAD(P)-abhängigen Dehydrogenase oxidiert wird und dadurch reduziertes NAD(P)H erzeugt. Unter einer NAD(P)-abhängigen Dehydrogenase werden die Enzyme der Hauptgruppe EC 1.x.1.y verstanden. Beispiele für Analyten mit der zugehörigen Dehydrogenase sind Lactat/Lactatdehydrogenase, Äthanol/Alkoholdehydrogenase, Glucose/Glucosedehydrogenase, Pyruvat/Pyruvatdehydrogenase u. a.

Unter einem chromogenen Redox-Indikator für NAD(P)H werden Substanzen verstanden, die Elektronen von NAD(P)H unter Enzymkatalyse direkt übernehmen können und dann in ihrer reduzierten Form eine Farbe im visuellen Bereich aufweisen, die verschieden ist von der Farbe der oxidierten Form. Bevorzugt liegt das Absorptionsmaximum der oxidierten Form des Redox-Indikators dabei im nicht-sichtbaren Bereich oder im Bereich blauen Lichts (≤ 500 nm) und die Farbe der reduzierten Form in Wellenlängenbereich zwischen 500 und 700 mm.

Die Nitrosoanilinverbindungen der Formel I fungieren in dem erfindungsgemäßen Verfahren in Anwesenheit von Benzylalkoholdehydrogenase gleichzeitig als direkte Elektronenakzeptoren des NAD(P)H/Benzylalkoholdehydrogenase-Systems und als chromogene Redox-Indikatoren. Unter direkten Elektronenakzeptoren wird verstanden, daß die bei der enzymatischen Oxidation von NAD(P)H freiwerdenden Elektronen enzymkatalysiert auf die Nitrosoanilinverbindung übertragen werden. Es hat sich herausgestellt, daß das Enzym Benzylalkoholdehydrogenase eine solche Reaktion sehr schnell und spezifisch katalysiert. Es wird vermutet, daß bei der enzymatischen Reduktion der Nitrosoanilinverbindung intermediär die entsprechende Hydroxylaminanilinverbindung entsteht, die dann spontan und sehr schnell Wasser abspaltet, so daß als Endprodukt der enzymatischen Reduktion der chromogenen Nitrosoanilinverbindungen der allgemeinen Formel I ein farbiges Chinondiimin der allgemeinen Formel II 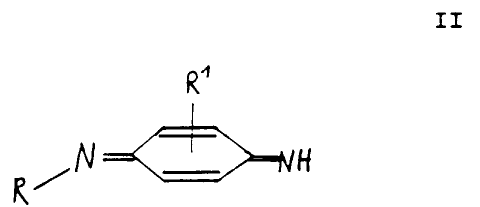 resultiert, das über seine Farbe in Form einer Intensitätsmessung direkt mit der Konzentration an NAD(P)H beziehungsweise eines Analyten in der Probenlösung korreliert werden kann.

Der farberzeugende Rest R der Verbindungen der allgemeinen Formel I und II stellt vorzugsweise einen elektronenreichen aromatischen oder heteroaromatischen Rest dar, dessen Elektronen mit einem elektronenärmeren System, wie es insbesondere der Imingruppen-tragende Teil der allgemeinen Formel II darstellt, in Konjugation treten können, so daß ein farbiges Molekül vom allgemeinen Typ der Polymethinfarbstoffe entsteht.

Es steht eine große Anzahl solcher farberzeugenden Reste R zur Verfügung. Denn farbige Chinondiimine der allgemeinen Formel II sind an sich sehr gut bekannt: sie entstehen auf anderem Wege durch oxidative Kupplung von farberzeugenden Kupplungsreagenzien R-H mit p-Phenylendiaminderivaten. Diese oxidative Kupplung ist insbesondere aus der Farbfotografie bekannt (zum Beispiel T. H. James, "The Theory of the Photographic Process", 3rd ed. Mc Millan, New York, 1966, Chapter 17 ("Principles and Chemistry of Color Photography") Seite 382 - 396 oder Ullmann's Encyclopedia of Industrial Chemistry eth ed. Vol A20, Seite 72 - 74, Verlag Chemie, Weinheim. 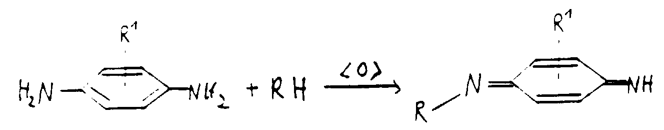 Eine weitere Anwendung erfährt die oxidative Kupplung von farberzeugenden Kupplungsreagenzien mit p-Phenylendiaminderivaten zur Herstellung farbiger Chinondiimine der allgemeinen Formel II in Analysenverfahren zum Nachweis von oxidierenden Substanzen, wie zum Beispiel H₂O₂ oder Peroxidase, oder zum Nachweis von elektronenreichen Anilinverbindungen (siehe beispielsweise EP-A-175 250). Am gebräuchlichsten als solche farberzeugenden Kupplungsreagenzien sind Phenole, Phenolderivate, Naphthol, Naphtholderivate, Naphthylamin, Naphthylaminderivate, Anilinderivate. Gebräuchlich sind auch methylenaktive und heterocyclische Verbindungen.

Zur Darstellung farbiger Chinondiimine der allgemeinen Formel II ist auch noch die Möglichkeit bekannt, heteroaromatische Amine R-NH2, wie zum Beispiel 3-Aminopyrazolo-Heterozyklen oder Pyrazolone wie 4-Aminoantipyrin, mit deren Aminogruppe an Anilinderivate zu einem farbigen Chinondiimin der allgemeinen Formel II oxidativ zu kuppeln (siehe EP-A-0 433 854).

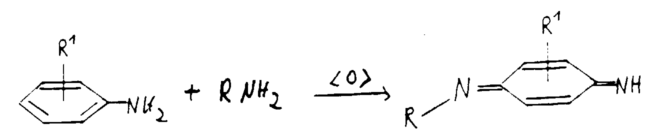

Die farberzeugenden Reste R aller dieser über oxidative Kupplungsreaktionen zu farbigen Chinondiiminverbindungen der Formel II führenden Kupplungsverbindungen RH und R-NH₂ sind als Reste R in den erfindungsgemäß einsetzbaren Nitrosoanilinverbindungen der Formel I ebenfalls geeignet. Voraussetzung ist lediglich, daß sie über ihr Elektronensystem mit der Iminogruppe der Formel II wechselwirken und dadurch eine Farbe in sichtbarem Bereich hervorrufen. Der Fachmann kann ohne Aufwand geeignete Reste für eine von ihm gewünschte Wellenlänge aussuchen und entsprechend substituierte Nitrosoanilinverbindungen in dem erfindungsgemäßen Verfahren einsetzen. Für die enzymatische Reduktion des Nitrosoanilinteils bleibt der Rest R ohne wesentlichen Einfluß. Trotz der Vielzahl der möglichen Reste werden die Nitrosoanilinverbindungen der Formel I von der Benzylalkoholdehydrogenase als Substrat und Elektronenakzeptor akzeptiert.

Bevorzugte Reste R sind Anilinreste, Naphthylaminreste, Phenol- und Naphtholreste, die weiter Substituenten tragen können, insbesondere die Reste der allgemeinen Formel III. 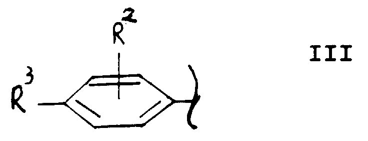 in der
- R²: Wasserstoff Hydroxy, Halogen, Alkoxy, Alkylthio, Aryloxy oder Arylthio, Alkyl oder Alkylen, gegebenenfalls substituiert durch Carboxy, P0₃H₂ oder SO₃H, Amino, gegebenenfalls ein- oder zweimal substituiert durch Alkyl, das gegebenenfalls wiederum durch Hydroxy, P0₃H₂, SO₃H, oder Carboxy substituiert sein kann, bedeutet oder mit dem Rest R¹ eine -CH₂-CH₂-, CH₂-0-,-CR¹² = CR¹³-, -0-, -S-, oder -NH- Brücke bilden kann, wobei -NH- gegebenenfalls durch einen Alkylrest substituiert sein kann und R¹² und R¹³ Carboxy oder Alkyl bedeutet, das gegebenenfalls durch Hydroxy, Carboxy, P0₃H₂ oder SO₃H substituiert sein kann.
- R³: eine Hydroxygruppe, Alkyl-, Alkoxy-, Aryl-, Aryloxy-, Arylthio- oder Alkylthiogruppe bedeutet, wobei der Alkylrest gegebenenfalls seinerseits durch eine Hydroxygruppe, eine Alkoxygruppe, eine gegebenenfalls ein- oder mehrfach durch Alkyl substituierte Aminogruppe, P0₃H₂, SO₃H oder CO₂H als solche oder in Salzform als Ammonium-, Alkali- oder Erdalkalisalze substituiert ist,
oder eine Aminogruppe NR⁴R⁵ bedeutet
in der R⁴ und R⁵ gleich oder verschieden sein können, und Wasserstoff, eine Alkylgruppe, die ihrerseits durch eine Hydroxy-, Alkoxy-, Hydroxyalkoxy-, eine gegebenenfalls hydroxysubstituierte Polyalkoxygruppe P0₃H₂, S0₃H, COOH als solche oder in Salzform oder durch eine gegebenenfalls ein- oder mehrfach durch Alkyl substituierte Aminogruppe substituiert sein kann, bedeutet, oder
in der R⁴ und R⁵ einen Alkylenrest darstellen können, der gegebenenfalls durch Sauerstoff, Schwefel oder Stickstoff unterbrochen ist, wobei Stickstoff durch einen Alkyl-, Hydroxyalkyl-, Hydroxyalkoxyalkyl-, Alkoxyhydroxyalkyl-, Alkoxycarbonylalkyl-, Dioxanylylalkyl oder Polyalkoxyalkylrest substituiert ist, der seinerseits jeweils gegebenenfalls im Alkylteil durch einen Hydroxyrest substituiert sein kann, oder wenn R² in ortho-Stellung zu NR⁴R⁵ steht, R⁴ oder R⁵ auch zusammen mit R² einen Alkylenrest darstellen kann.
Hierbei bedeutet Halogen Fluor, Chlor, Brom oder Jod. Fluor und Chlor sind besonders bevorzugt.
Alkyl in Alkyl, Alkoxy oder Alkylthio bedeutet einen Kohlenwasserstoffrest mit 1 - 6 Kohlenstoffatomen, Reste mit 1 - 3 Kohlenstoffatomen sind besonders bevorzugt. Die für Alkyl vorstehend gegebene Definition trifft auch für den Alkylteil in Hydroxyalkyl-, Dialkylaminoalkyl-, Hydroxyalkoxyalkyl-, Alkoxyalkyl-, Polyalkoxyalkyl-, Alkoxy-hydroxyalkyl- und Dioxanylyl-alkylresten zu. Ein Dioxanylyl-alkylrest ist ein Rest, bei dem ein Dioxanringsystem an einen Alkylrest gebunden ist. Vorzugsweise handelt es sich um ein 1,4-Dioxanringsystem, d. h. 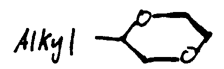 Ein Polyalkoxyalkylrest ist ein Rest -Alkyl-(Alkoxy)ₙ-Alkoxy
mit n = 1 - 10. Bevorzugt ist n = 1 - 4. Besonders bevorzugt ist n = 1 - 3. Ein Alkylenrest ist eine geradkettige oder verzweigte - vorzugsweise geradkettige -, gesättigte oder ungesättigte - vorzugsweise gesättigte-, Kohlenwasserstoffkette aus 2 - 5, vorzugsweise 2 - 4 C-Atomen, mit zwei freien Bindungsstellen. Aryl in Aryl- und Aralkylresten ist ein 6 bis 10 Kohlenstoffatome zählendes aromatisches Ringsystem, wobei Phenyl bevorzugt ist.
Ammoniumsalze sind solche, die das Ammoniumion NH₄+ enthalten oder solche, die ein- oder mehrfach durch Alkyl-, Aryl- oder Aralkylreste substituierte Ammoniumkationen enthalten.
Alkalisalze sind vorzugsweise solche des Lithium, Natriums oder Kaliums. Erdalkalisalze sind vorzugsweise solche des Magnesiums oder Calciums.
In der Bedeutung eines von R² und R⁴ durch Sauerstoff, Schwefel oder Stickstoff unterbrochenen Alkylenrestes ist der durch Einbeziehung des Stickstoffatomes der allgemeinen Formel I gebildete Morpholin-bzw. Thiomorpholin- bzw. Piperazinrest bevorzugt. Der Piperazinrest ist besonders bevorzugt.
In der Bedeutung eines von R² und R⁴ gebildeten Alkylenrestes ist der durch Einbeziehung des aromatischen Ringes der allgemeinen Formel I gebildete Indolin - oder 1,2,3,4-Tetrahydrochinolinrest bevorzugt.
Als Salz eines erfindungsgemäßen Nitrosoanilinderivates der allgemeinen Formel I werden insbesondere solche starker Säuren, insbesondere Mineralsäuren, wie Salzsäure, Schwefelsäure, Salpetersäure, Phosphorsäure bevorzugt. Ganz besonders bevorzugt sind Hydrochloride, das sind Salze der Salzsäure.

Für R² ist Wasserstoff für R³ die Hydroxy, Alkoxy- und die disubstituierte Aminogruppe NR⁴R⁵ bevorzugt.

Besonders bevorzugt sind als Reste R der Formel III N,N-disubstituierte Anilinreste wie N, N-Dihydroxyethylanilin, N,N-Diethylanilin, N,N-Dimethylanilin.

Geeignet als farberzeugende Reste R sind auch die aus der Farbfotografie bekannten CH-aciden Verbindungen wie 1,3 Indandion, Thioindoxyl oder Indoxyl sowie methylenaktive heterocyclische Verbindungen wie Pyrazolone, u.ä. Bevorzugt unter den heteroaromatischen chromogenen Resten sind Pyrazole, Pyrazolone und Pyrazoloverbindungen, wie sie zum Beispiel aus Ullmann's Encyclopedia of Industrial Chemistry, 5th ed., Vol. A20, Seite 72 - 74, Verlag Chemie, Weinheim, bekannt sind, insbesondere der Antipyrinrest und anellierte Pyrazoloreste der Formel IV, wie sie größtenteils als 3-aminosubstituierte Verbindungen in EP-A-0433 854 beschrieben sind.

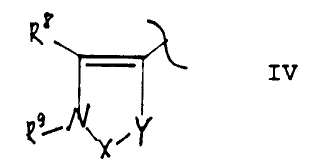

Dabei bedeutet:
- X-Y: NR⁶-CO, oder N=CR⁷
- R⁶: H, Alkyl gegebenenfalls substituiert durch Hydroxy, Carboxy, SO₃H, PO₃H₂, Dialkylphosphinyl
- R⁷: H, Alkyl, Alkenyl, Alkoxy, Alkylthio, Aryl, Aralkyl, gegebenenfalls jeweils substituiert durch Hydroxy, Dialkylphosphinyl, Carboxy, SO₃H, PO₃H₂ ein Salz eines dieser Säurereste oder / und Alkoxycarbonyl; oder
Amino, das gegebenenfalls durch einen oder zwei gegebenenfalls einen oder mehrere Hydroxy-, Carboxy- oder / und Alkoxycarbonylreste tragende Alkylreste substituiert ist wobei, wenn Amino durch 2 Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen
sein kann oder Amino gegebenenfalls durch ein oder zwei Acylgruppen, Alkoxy- oder / und Aralkoxy-Carbonylgruppen, H₂N-CO, Alkyl, Aralkyl oder / und Aryl-Carbamoylgruppen substituiert ist; oder Wasserstoff, Carboxy, Alkoxycarbonyl, Carboxamido oder Halogen ist und
- R⁸: Alkyl, Thioalkyl oder Aralkyl, gegebenenfalls substituiert durch Hydroxy, Carboxy, SO₃H oder PO₃H₂ oder Amino, das gegebenenfalls durch eine oder zwei Alkylgruppen substituiert ist, die wiederum durch Hydroxy, Carboxy, SO₃H, Dialkylphosphinyl oder PO₃H₂ substituiert sein können,
wobei mindestens R⁷ und / oder R⁸ eine Aminogruppe darstellt, und
- R⁹: eine Alkyl oder Aralkylgruppe, die gegebenenfalls durch Hydroxy, Carboxy, SO₃H oder PO₃H₂ substituiert sein kann
oder wobei
R⁸ und R⁹ zusammen eine gesättigte oder ungesättigte Kette mit 3 oder 4 Gliedern aus Stickstoffatomen oder aus Kohlenstoffatomen und gegebenenfalls einem oder mehreren Stickstoff- oder Schwefelatomen bilden können, wobei Kohlenstoffatome gegebenenfalls substituiert sind durch Alkyl, Alkoxy, Alkylthio, Hydroxy, Aralkyl, Aryl, Carboxy, Carboxamido, Alkoxycarbonyl, Cyano, Halogen, Amino, das gegebenenfalls durch einen oder zwei gegebenenfalls einen oder mehrere Hydroxy-, Carboxy- oder / und Alkoxycarbonylreste tragende Alkylreste substituiert ist, und wobei Stickstoffatome, die nicht über eine Doppelbindung gebunden sind, durch Wasserstoff, Alkyl, gegebenenfalls durch Hydroxy, SO₃H, PO₃H₂, Carboxy oder Dialkylphosphinyl substituiert oder durch Aralkyl, substituiert sind oder zwei benachbarte Kettensubstituenten
gegebenenfalls eine Alkylengruppe bilden, die ihrerseits gegebenenfalls mit Aryl substituiert oder anelliert ist
sowie gegebenenfalls entsprechende tautomere Formen und deren Salze

Hierbei bedeutet "Alkyl" - auch in Alkylthio-, Dialkylphosphinyl-, Alkylcarbamoyl- und Aralkylresten - einen geradkettigen oder verzweigten Alkylrest mit 1-6, vorzugsweise 1-4 C-Atomen. Beispiele sind die Methyl-, Ethyl-, Propyl-, iso-Butyl- oder tert.-Butylgruppe.

Wenn eine Aminogruppe durch 2 Alkylreste substituiert ist, können diese Reste auch so zu einem Ring geschlossen sein, daß sie insgesamt einen durch ein Stickstoffatom unterbrochenen Ring darstellen. Bevorzugt sind hierbei solche Aminogruppen, die einen insgesamt 5- oder 6-gliedrigen Ring darstellen, der seinerseits gegebenenfalls durch Sauerstoff, Schwefel oder Stickstoff unterbrochen ist. Besonders bevorzugt ist der Morpholinorest.

"Alkoxy" - auch in Alkoxy- und Aralkoxycarbonylresten steht für einen geradkettigen oder verzweigten Alkoxyrest mit 1-6, vorzugsweise 1-4 C-Atomen. Beispiele sind die Methoxy-, Propyloxy-, iso-Butyloxy oder tert.-Butyloxygruppe.

"Aryl" - auch in Arylcarbamoylgruppen - bezeichnet einen Kohlenstoffaromaten - oder Heteroaromatenrest, vorzugsweise einen solchen mit 6-10 Ring-Atomen, insbesondere die Phenyl- und die Naphthylgruppe, die zusätzlich noch durch Alkyl, Alkoxy oder / und Halogen substituiert sein können. Besonders bevorzugt ist der Phenylrest.

Ein "Aralkyl"-Rest - auch in einer Aralkylcarbamoylgruppe -bedeutet einen Rest, bei dem eine wie vorstehend definierte Alkylengruppe durch einen wie zuvor charakterisierten Arylrest substituiert ist. Bevorzugt ist die Benzylgruppe.

Ein "Aralkoxy"-Rest, beispielsweise in Aralkoxycarbonylgruppen, bezeichet einen Rest, bei dem eine wie vorstehend definierte Alkoxygruppe durch einen wie zuvor charakterisierten Arylrest substituiert ist. Bevorzugt ist die Benzyloxygruppe.

"Halogen" steht für die Reste Fluor, Chlor, Brom und Jod. Fluor und Chlor sind bevorzugt.

Eine Acylgruppe bezeichnet einen Carbonsäurerest, der Alkyl-, Aralkyl- oder Arylreste enthalten kann. Bevorzugt sind Acetyl-, Phenylacetyl oder Benzoylreste.

Unter einer Alkylengruppe wird eine geradkettige oder verzweigte, gesättigte oder ungesättigte Kohlenwasserstoffkette aus 3-4 C-Atomen mit zwei freien Bindungsstellen verstanden.

Beispiele sind -CH₂-CH=CH, 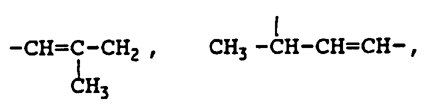 -(CH₂)₄ - oder -CH=CH-CH=CH-

Bevorzugt sind der Butadiendiylrest (-CH=CH-CH=CH-) und der Tetramethylenrest (-(CH₂)₄ - ).

Ein Alkenyl ist ein geradkettiger oder verzweigter Kohlenwasserstoffrest aus 2-5 C-Atomen mit mindestens einer Doppelbindung. Bevorzugt ist beispielsweise der Vinylrest. Unter einer Dialkylphosphinylgruppe wird der Rest 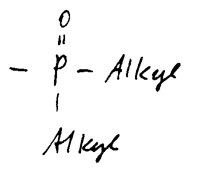 verstanden, wobei Alkyl die zuvor gegebene Bedeutung hat. Bevorzugt ist der Dimethylphosphinylrest.

Als Salze von SO₃H, PO₃H₂ und Carboxyreste können Alkali- oder Erdalkalisalze oder Ammoniumsalze eingesetzt werden. Unter Alkalisalzen werden Lithium-, Natrium-, Kalium-, Rubidium- oder Cäsiumssalze verstanden, wobei Lithium-, Natrium- und Kaliumsalze, vor allem aber Natrium- und Kaliumsalze bevorzugt sind. Erdalkalisalze sind solche des Berylliums, Magnesiums, Calciums, Strontiums oder Bariums. Bevorzugt sind Magnesium- und Calciumsalze, wobei Calciumsalze besonders bevorzugt sind. Als Ammoniumsalze können solche des unsubstituierten Ammoniums, NH₄⁺, Verwendung finden. Es ist aber auch möglich, solche Ammoniumsalze einzusetzen, bei denen das Ammoniumion durch 1-4 Alkyl, Aryl- oder Aralkylreste substituiert ist. Für diese Reste gelten die zuvor gegebenen Definitionen, wobei als Alkylrest Methyl, Ethyl und n-Propyl, als Arylrest die Phenylgruppe und als Aralkylrest die Benzylgruppe besonders bevorzugt sind.

Als Carboxamidorest wird der Rest CONH₂ verstanden, aber auch solche Reste, bei denen die Aminogruppe durch ein oder zwei gegebenenfalls einen oder mehrere Hydroxy-, Carboxy- oder / und Alkoxycarbonylreste tragende Alkylreste substituiert ist.

In den erfindungsgemäß eingesetzten Nitroso-Verbindungen der allgemeinen Formel I bilden R⁸ und R⁹ bevorzugt einen gesättigten oder ungesättigten Ring. Dabei sind solche Ringe besonders bevorzugt, in denen Doppelbindungselektronen und freie Stickstoffelektronenpaare der ungesättigten Kette in Konjugation zur Doppelbindung oder zu dem Brücken-N-Atom der allgemeinen Formel I stehen, so daß ein anellierter aromatischer Ring resultiert.

Gegebenenfalls sind für einen farberzeugenden Rest der allgemeinen Formel IV auch tautomere Formen möglich. Diese sollen ebenfalls als von der allgemeinen Formel IV umfaßt gelten.

Erfindungsgemäß bevorzugt sind farberzeugende Reste der allgemeinen Formel V bis XIV. 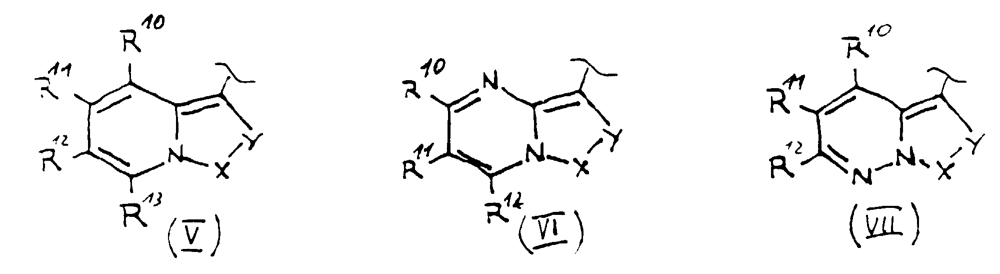 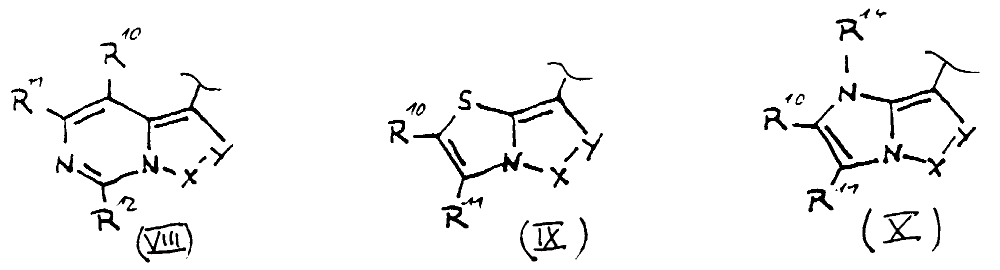 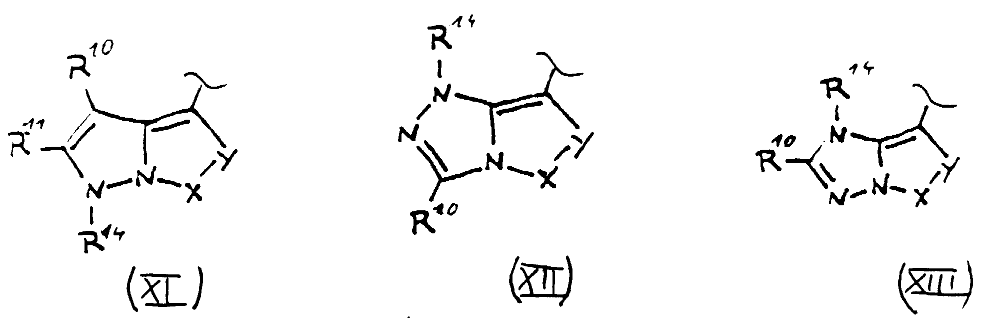 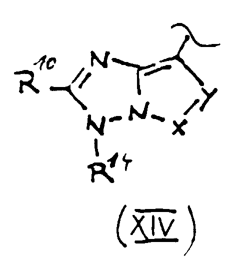 sowie entsprechende tautomere Formen und deren Salze.

Hierbei haben X-Y die gleiche Bedeutung wie zuvor beschrieben. R¹⁰, R¹¹, R¹² und R¹³, die gleich oder verschieden sind, stehen für Wasserstoff, Hydroxy, Alkyl, Alkoxy, Alkylthio, Aralkyl, Aryl, Carboxy, Alkoxycarbonyl, Carboxamido, Cyano, Amino, das gegebenenfalls durch ein oder zwei gegebenenfalls einen oder mehrere Hydroxy-, Carboxy- oder / und Alkoxycarbonylreste tragende Alkylreste substituiert ist oder Halogen, wobei zwei benachbarte Reste gegebenenfalls eine Alkylengruppe bilden, die ihrerseits gegebenenfalls mit Aryl substituiert oder anelliert ist und R¹⁴ Alkyl oder Aralkyl darstellt, das gegebenenfalls durch Hydroxy, Carboxy, SO₃H, PO₃H₂ oder Dialkylphosphinyl substituiert ist. Die Definition der Reste entsprechen den für die Reste der allgemeinen Formel IV gegebenen.

Besonders bevorzugt für die erfindungsgemäße Verwendung sind Reste der allgemeinen Formel V, VI, VII, IX, X und XI,
gegebenenfalls entsprechende tautomere Formen und deren Salze. Ganz besonders bevorzugt sind solche Substanzen, in denen X-Y die Bedeutung N=CR⁶ hat, wobei R⁶ die Bedeutung haben kann wie für die allgemeine Formel IV angegeben. Als hervorragend geeignet für die erfindungsgemäße Verwendung haben sich insbesondere folgende Verbindungen erwiesen.
1) (2-Methyl-pyrazolo [1.5a]-pyridin-3-yl)-(4' nitrosophenyl)-amin
2) (Pyrazolo-[1.5a]-pyridin-3-yl)-(4'nitrosophenyl)-amin
3) (2-Methoxy-pyrazolo [1.5a] pyridin-3-yl)-(4'nitrosophenyl)-amin
4) (2-Hydroxyethyl-pyrazolo [1.5a] pyridin-3-yl)-(4'nitrosophenyl)-amin
5) (2,4 Dimethyl-pyrazolo-[1.5a]-imidazol-3-yl)-(4'nitrosophenyl)-amin
6) 4-Methyl-pyrazolo-[1.5a]-imidazol-3-yl)-(4'nitrosophenyl)-amin
7) (2-Methyl-4-dimethylphosphinylmethyl-pyrazolo [1.5a] imidazol-3-yl)-(4'-nitrosophenyl)-amin
8) (2-Methyl-4-sulfopropyl-pyrazolo-[1.5a] imidazol-3-yl)-(4'nitrosophenyl)-amin
9) (2-Sulfopropyl-pyrazolo-[1.5a] pyridin-3-yl)-(4'nitrosophenyl)-amin
10) (Pyrazolo [1.5a] pyrimidin-3-yl)-(4'nitrosophenyl)-amin
11) (2-Methyl-4-dimethylphosphinylmethyl-pyrazolo-[1.5a] benzimidazol-3-yl)-(4-nitrosophenyl)-amin
12) (2-Methyl-pyrazolo-[1.5a]-pyridin-3-yl)-4-nitroso-2-sulfopropyl-phenyl)-amin
13) (2-Methyl-pyrazolo-[1.5a] pyridin-3-yl)-4-(nitroso-2-hydroxypropyl-phenyl)-amin
14) (2-Methyl-5,6-dihydro-4-dimethylphosphinylmethylpyrazolo-[1.5a] imidazol-3-yl)-(4'nitrosophenyl)-amin

Ganz besonders bevorzugt sind die Verbindungen Nr. 2,3,6,7,8,9,12 und 13.
Die eingesetzte Benzylalkoholdehydrogenase kann aus verschiedenen Organismen (Acinetobakter-, Pseudomonas-, Rhodopseudomonas-Stämmen und andere) erhalten werden.

Wie vorstehend ausgeführt, werden für das erfindungsgemäße Verfahren Nitrosoanilinverbindungen der Formel I mit der auf ihren NAD(P)H-Gehalt zu untersuchenden Probe und Benzylalkoholdehydrogenase in Kontakt gebracht. Enthält die Probe NAD(P)H oder einen Analyten, durch dessen Umsetzung mit NAD-abhängigen Dehydrogenasen NAD(P)H entsteht, so reagiert die Nitrosoanilinverbindung in Anwesenheit der Benzylalkoholdehydrogenase zum entsprechenden farbigen Chinondiimin der allgemeinen Formel II unter Reoxidation des NAD(P)H zu NAD(P)+. Die Farbe der Chinondiiminverbindient als Maß für die Menge des NAD(P)H und damit gegebenenfalls auch für die Menge des Analyten. Die Farbmessung kann direkt visuell, eventuell über Vergleichsfarben, oder photometrisch erfolgen.

Das erfindungsgemäße Verfahren bietet den Vorteil, daß NAD(P)H unter Enzymkatalyse schnell und spezifisch in einer Stufe nachgewiesen werden kann. Insbesondere sind die eingesetzten chromogenen Redox-Indikatoren gegen Basen und Licht stabil und liefern bei Reduktion direkt intensive Farben wählbar über den gesamten sichtbaren Wellenlängenbereich. Unspezifische Redoxkatalysatoren wie Diaphorase oder N-Methylphenaziniumsulfat werden nicht benötigt.

Selbstverständlich eignen sich die erfindungsgemäßen Nitrosoanilinverbindungen auch dazu, die Anwesenheit oder die Aktivität von NAD(P)⁺-abhängigen Dehydrogenasen oder von Benzylalkoholdehydrogenase zu bestimmen.

Soll Benzylalkoholdehydrogenase bestimmt werden, wird NAD(P)H und eine chromogene Nitrosoanilinverbindung der Formel I mit einer Benzylalkoholdehydrogenase enthaltenden Probe umgesetzt und die Farbe der entstehenden Chinondiiminverbindung gemessen. Die zeitliche Änderung der Farbe kann mit der Enzymaktivität korreliert werden.

Soll eine NAD(P)⁺-abhängige Dehydrogenase bestimmt werden, wird eine Probe, die auf diese Dehydrogenase getestet werden soll, mit einem Substrat dieser Dehydrogenase, NAD(P)H, Benzylalkoholdehydrogenase und einer chromogenen Nitrosoanilinverbindung der Formel I umgesetzt und die Farbe des entstehenden Chinondiimins gemessen.

Ein weiterer Gegenstand der Erfindung ist ein Mittel zur kolorimetrischen Bestimmung von NAD(P)H, bestehend aus einem NAD(P)H-oxidierenden Enzym und einem direkten chromogenen Elektronenakzeptor, dadurch gekennzeichnet, daß es Benzylalkoholdehydrogenase und eine Nitrosoanilinverbindung der allgemeinen Formel I enthält.

Weiter ist Gegenstand der Erfindung ein Mittel zur kolorimetrischen Bestimmung von Analyten, durch deren enzymatische Oxidation NAD(P)H gebildet wird, das neben Benzylalkoholdehydrogenase und einer Nitrosoanilinverbindung der allgemeinen Formel I eine analytspezifische, NAD(P)+-abhängige Dehydrogenase zusammen mit ihrem Cofaktor enthält.

Als Benzylalkoholdehydrogenasen, Nitrosoanilinverbindungen und gegebenenfalls analytspezifische Dehydrogenasen werden die vorstehend für das erfindungsgemäße Verfahren beschriebenenen Stoffe eingesetzt.

Zur Einhaltung eines für die Durchführung des Verfahrens geeigneten pH-Wertes, der sich primär nach der einzusetzenden Benzylalkoholdehydrogenase, sekundär gegebenenfalls auch nach den eingesetzten NAD-abhängigen Dehydrogenasen richtet, enthält das erfindungsgemäße Mittel ein Puffersystem. Vorteilhafterweise enthält das Mittel ein Puffersystem, das in der Testlösung einen pH-Wert zwischen pH 6 und 9 einstellt. Besonders vorteilhaft ist ein pH-Wert zwischen 7 und 8,5.

Das erfindungsgemäße Mittel kann in Form einer Lösung oder auf einem saugfähigen oder quellbaren Träger aufgezogen vorliegen. In Form einer Lösung enthält das Mittel vorzugsweise sämtliche für das erfindungsgemäße Verfahren benötigten Reagenzien. Als Lösungsmittel kommt vorzugsweise Wasser oder Mischungen aus Wasser mit wasserlöslichen organischen Lösungsmitteln, wie zum Beispiel Methanol, Äthanol, Aceton oder Dimethylformamid in Frage. Aus Haltbarkeitsgründen kann es vorteilhaft sein, die für den Test benötigten Reagenzien auf zwei oder mehrere Lösungen zu verteilen, die erst bei der eigentlichen Untersuchung vermischt werden.

Die Konzentration der eingesetzten aromatischen Nitrosoverbindungen richtet sich nach der Konzentration des zu messenden NADH beziehungsweise des zu messenden Analyten. Typische Konzentrationen für die im erfindungsgemäßen Verfahren eingesetzten Nitrosoverbindungen sind 0,01 - 100 mmol/l, bevorzugt 0,1 - 25 mmol/l. Die Konzentration der eingesetzten Benzylalkoholdehydrogenase richtet sich nach der Konzentration des Analyten und der gewünschten Analysezeit. Typische Werte für Enzym-Konzentrationen sind 1mU bis 10U/ml bei Küvettentests.

Das erfindungsgemäße Mittel kann auch in Form eines Teststreifens vorliegen. Solche Teststreifen sind in verschiedensten Ausführungsarten bekannt, zum Beispiel aus EP-A-0 016 387, DE-A-3 247 608, EP-A-0 262 445 oder EP-A-0 256 806. In einem Teststreifen liegen die zur Durchführung des Bestimmungsverfahrens benötigten Reagenzien auf festen Trägerschichten vor, als Trägerschichten kommen insbesondere saugfähige und/oder quellbare Materialien in Frage, die von der zu untersuchenden Probenflüssigkeit benetzt werden. Beispiele sind Gelatine-, Zellulose- oder Kunstfaservliese. In oder auf diesen Trägermaterialien liegen die Reagenzien in fester Form vor. Bei Aufgabe der Probenflüssigkeit auf den Teststreifen oder Eintauchen des Teststreifens in die Probenflüssigkeit bildet sich in den Streifen ein flüssiges Milieu aus, innerhalb dessen die Nachweisreaktion abläuft. Die durch die Reaktion verursachte Farbbildung kann visuell oder photometrisch, zum Beispiel reflexionsphotometrisch ausgewertet werden.

Typische Werte für Konzentrationen auf Teststreifen sind:

| | |
|---|---|
| Analyt | 10⁻⁴ - 10⁻¹ M |
| Nitrosoverbindung | 10⁻³ - 1 M |
| Enzym | 0,1 - 100 U pro |
| | Testfeld |

Chromogene Nitrosoanilinverbindungen der Formel I sind neu. Gegenstand der Erfindung sind daher Nitrosoanilinverbindungen der Formel I 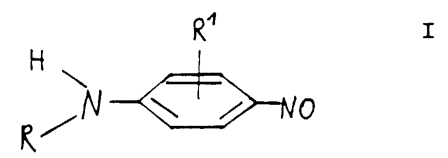 wobei
- R¹: Wasserstoff, Hydroxy, Halogen, C₁-C₆ Alkoxy oder Alkylthio, C₆-C₁₀ Aryloxy oder Arylthio, C₁-C₆ Alkyl oder Alkylen, gegebenenfalls substituiert durch Carboxy, PO₃H₂, Dialkylphosphinyl oder SO₃H, Amino, gegebenenfalls ein- oder zweimal substituiert durch C₁-C₆ Alkyl, das gegebenenfalls wiederrum durch Hydroxy, Carboxy, PO₃H₂ oder SO₃H substituiert sein kann, darstellt und
- R: einen bei Elektronen-Konjugation mit einem Chinondiimin-System farberzeugenden Rest darstellt,
Insbesondere sind Gegenstand der Erfindung die chromogenen Nitrosoanilinverbindungen der Formel I, in denen der chromogene Rest R einen Rest der allgemeinen Formel III oder IV darstellt.
Ein weiterer Gegenstand der Erfindung ist ein Verfahren zur Herstellung der Nitrosoanilinverbindung der Formel I.

Die Herstellung der erfindungsgemäßen Verbindungen erfolgt nach an sich bekannten Methoden, wie sie von J.T. Hays et al. im J. Org. Chemie 32, 158 (1967) beschrieben wurden. Dazu werden Ether, bevorzugt Methylether, von p-Nitrosophenolen der Formel XVII bevorzugt unter Protonenkatalyse mit Aminoverbindungen der Formel XVIII umgesetzt. Unter Substituion der Methoxygruppe entstehen sekundäre Amine der Formel I.

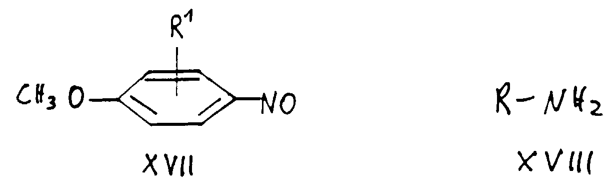

Die dazu benötigten Aryl- oder Hetarylamine sind entweder beschrieben oder können analog den bekannten Verfahren hergestellt werden. Aminoverbindungen, die als Grundbaustein Pyrazolo-Heterocyclen enthalten, sind in EP 0433854 beschrieben.

Die als Zwischenprodukt benötigten Aminoverbindungen liegen in der Regel aus Haltbarkeitsgründen als Salze von starken Säuren, z.B. Mineralsäuren vor. Zur Umsetzung mit p-Nitrosoanisolen werden bevorzugt die freien Basen der Aminoverbindungen eingesetzt, die man nach üblichen Methoden z.B. durch Auflösen der Salze in Wasser, Zugabe von Base bis zu einem pH von 8-10 und Extraktion der freien Base mit einem organischen LÖsungsmittel beispielsweise Essigester oder Methylenchlorid erhält. Alternativ dazu kann man, insbesondere bei schwer extrahierbaren Aminoverbindungen, folgendermaßen vorgehen: Man löst die Aminoverbindung in Methanol, gibt dann eine Base z.B. NaHCO₃-Lösung, Triethylamin usw. zu, bis der pH-Wert der Methanollösung auf einem nassen pH-Papier etwa den Wert 5-6 erreicht hat. Anschließend setzt man den zweiten Reaktionspartner, das p-Nitrosoanisol, zu.

### Beispiel 1

### NADH-Bestimmung mit chromogenen p-Nitrosoanilinen

In einer 1 cm Küvette wird folgender Ansatz vorgelegt:
100 mM Phosphatpuffer pH 7,
100 µM p-Nitrosoanilin der Formel 2 aus Tabelle 1
1 U/ml Benzylalkoholdehydrogenase aus Acinetobater

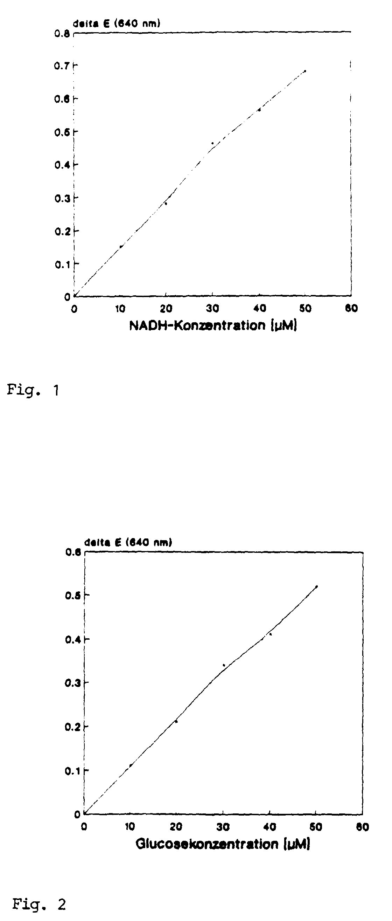

Unterschiedliche Mengen an NADH werden so zugegeben, daß die Küvettenlösung eine NADH-Konzentration zwischen 10 µmol/l und 50 µmol/l enthält. 5 Minuten nach Reaktionsstart wird die Extinktion der nun farbigen Lösungen bei 640 nm bestimmt (Figur 1). Mit der so erhaltenen Funktionskurve lassen sich unbekannte NADH-Mengen über Extinktionsmessungen bestimmen.

### Beispiel 2

### Glukosebestimmung

In einer 1 cm Küvette wird folgender Ansatz vorgelegt:
100 mM Phosphatpufer pH 7
100 µM p-Nitrosoanilin der Formel 2 in Tabelle 1
1 mM NAD+
1 U/ml Benzylalkoholdehydrogenase aus Acinetobater
5 U/ml Glukosedehydrogenase (Merck, Darmstadt, BRD)

Unterschiedliche Mengen an Glukose werden so zugegeben, daß die Kösung in der Küvette zwischen 10 und 50 µmol/l Glukosekonzentration enthält. 5 Minuten nach Reaktionsstart wird die Extinktion der nun farbigen Lösungen bei 640 nm bestimmt (Figur2). Mit der so erhaltenen Funktionskurve können unbekannte Glukosekonzentrationen durch Extinktionsmessung bestimmt werden.

### Beispiel 3

NADH bzw. Glukose wird entsprechend Beispiel 1 bzw. Beispiel 2 mit p-Nitrosoanilinverbindungen der Formeln 1 bis 18 in Tabelle I bestimmt. Tabelle I zeigt die Absorptionsmaxima der gebildeten farbigen Chinondiimine.

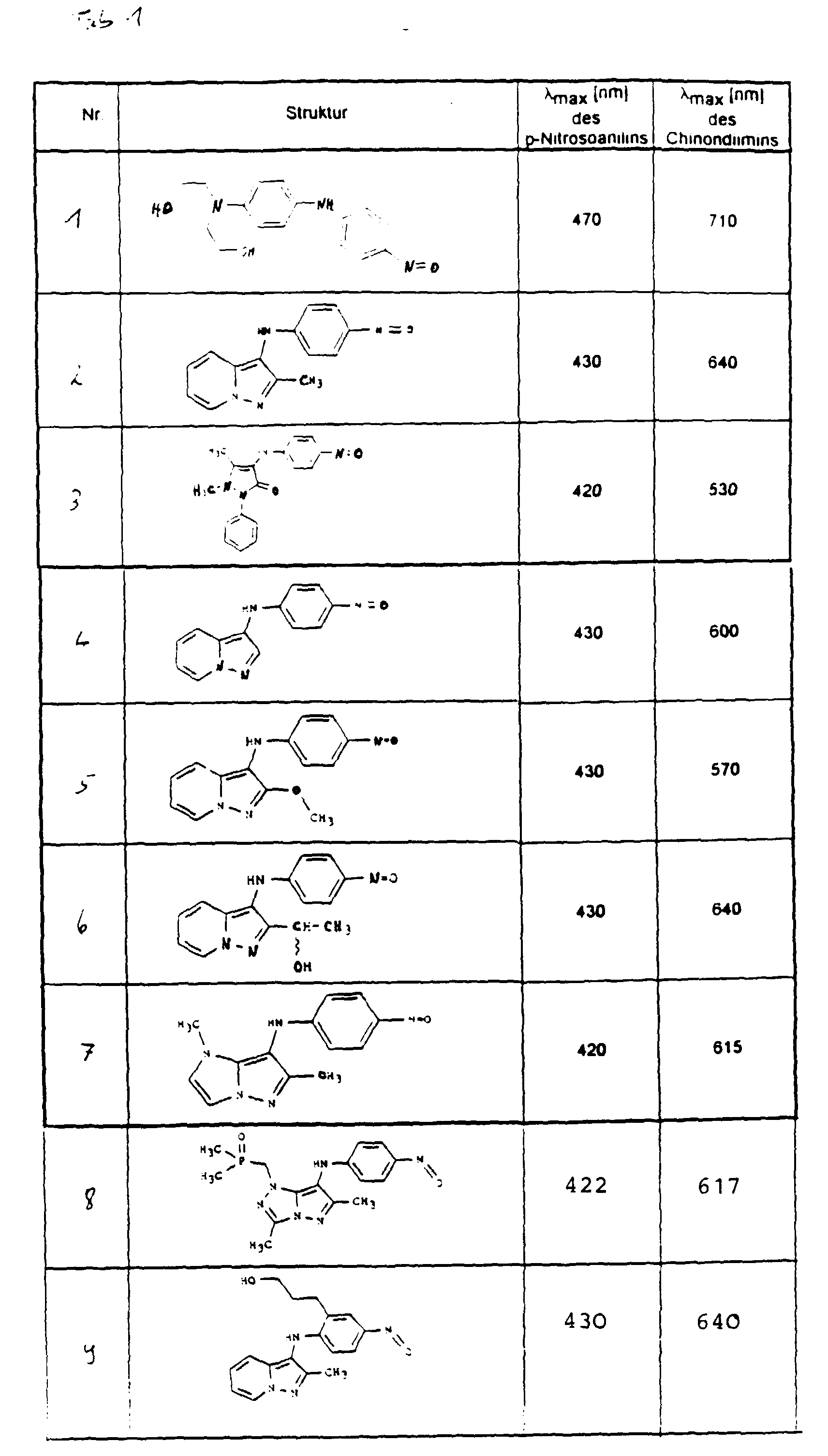

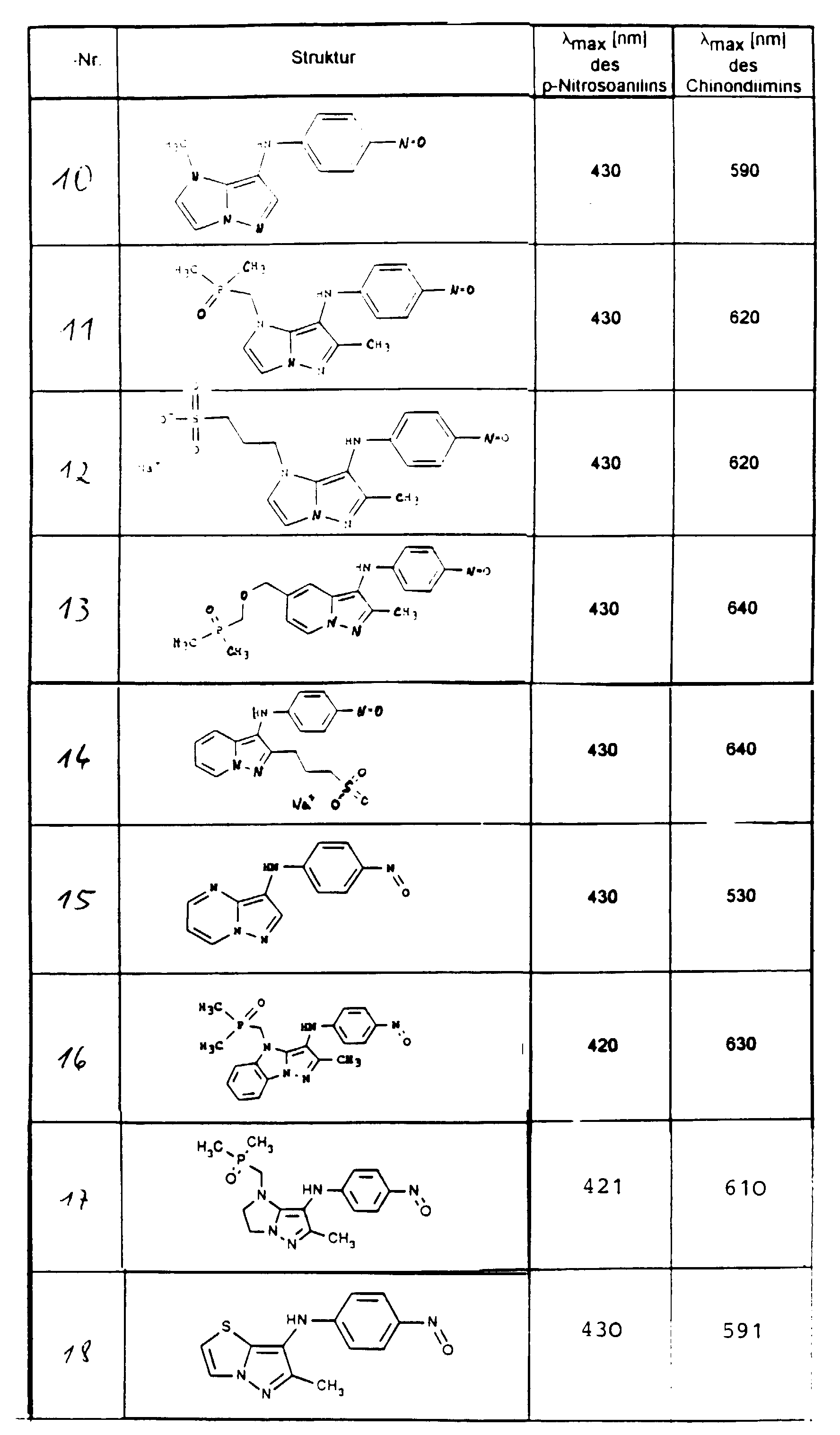

### Beispiel 4

### (2-Methyl-pyrazolo-[1.5a]-pyridin-3-yl)-(4'nitrosophenyl)-amin

0,8 g 3-Amino-2-methyl-pyrazolo-[1.5a]-pyridin-Hydrochlorid werden mit wässriger Natriumbicarbonatlösung in die freie Base überführt, die mit Methylenchlorid extrahiert wird. Der Rückstand des eingedampften Extraktes wird in 20 ml Methanol gelöst und mit 30 mg p-Toluolsulfonsäure versetzt. Bei 0-5° C gibt man dann eine Lösung von 0.54 g p-Nitrosoanisol in 10 ml Methanol zu. Die Reaktionsmischung wird 3 Stunden bei Raumtemperatur gerührt und eingedampft. Das Rohprodukt wird durch Chromatographie an Kieselgel mit dem Laufmittel Heptan/Methylethylketon 2:1 gereinigt. Man erhält 0,7g braune Kristalle vom Fp. 146° C.

DC (Kieselgel, Heptan/Methylethylketon 2:1) R_{f}=0.22

### Beispiel 5

Analog Beispiel 4 erhält man die in der Tabelle 2 aufgeführten Nitrosoverbindungen.

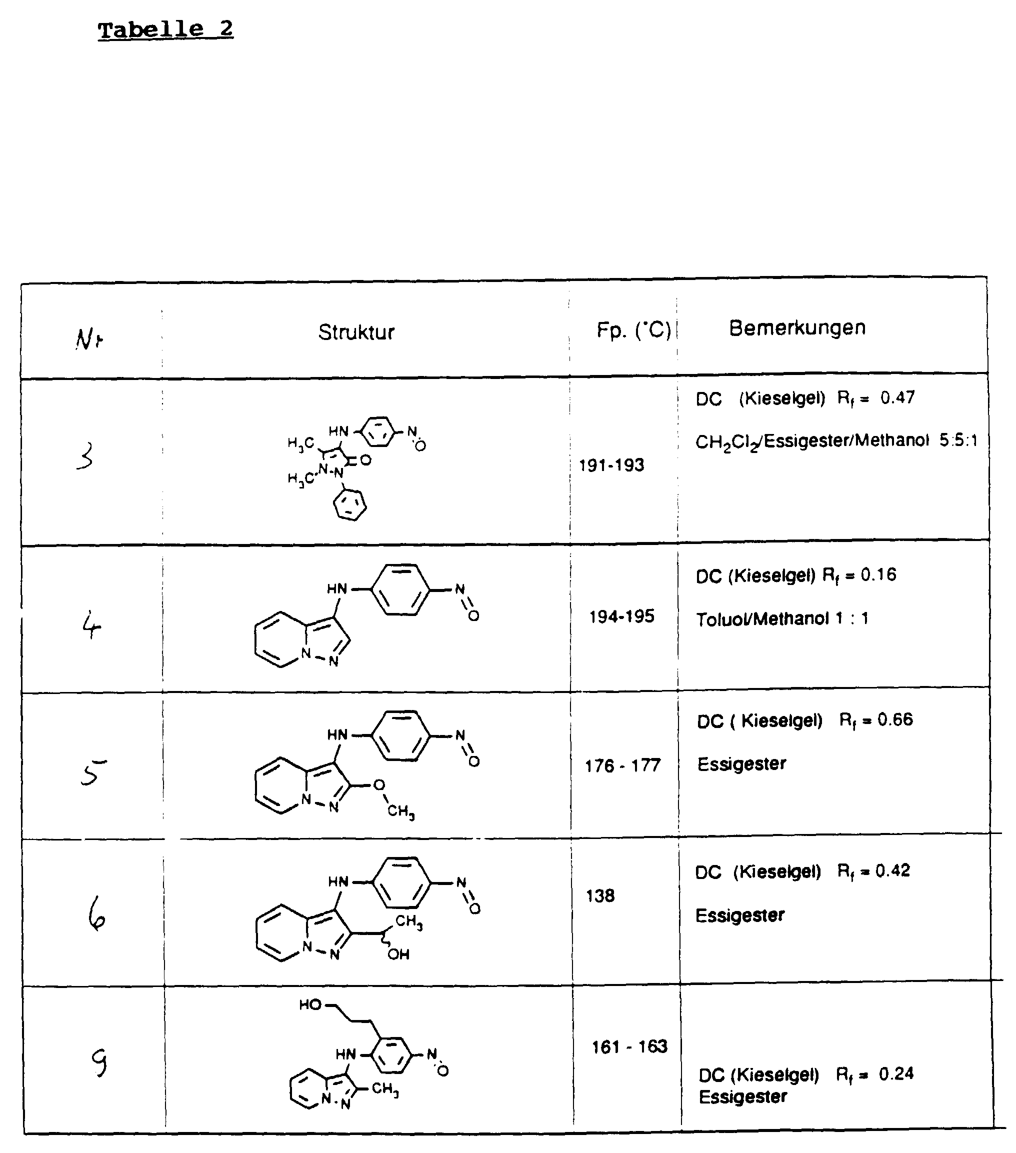

### Herstellung der Nitroso-Ausgangsverbindung zu Verbindung 9:

### 6-Nitroso-1,2,3,4-tetrahydro-benzopyran

7g 2-(3-Hydroxypropyl)phenol werden analog Bull. Soc. Chim. Fr. 337 (1957) in Wasser mit 3.5 g Natriumnitrit in Gegenwart von 3.0 g Aluminiumsulfat 18 H₂O nitrosiert. Die Suspension wird gut gerührt und nach 30 min. mit Essigester extrahiert. Der Rückstand aus dem Essigextrakt wird über Kieselgel mit Essigester/Ligroin 7:3 chromatographiert. Man erhält 3.8 g der entsprechenden p-Nitrosoverbindung. (DC(Kieselgel, Essigester/Ligroin 7:3) R_{f}=0.34), 2.6g davon werden in Methanol gelöst und unter Zusatz von ca 100 mg p-Toluolsulfonsäure 2 Std. unter Rückfluß gekocht. Die Reaktionsmischung wird eingeengt und durch Chromatographie über Kieselgel mit Essigester/Ligroin gereinigt. Man erhält 1 g der Titelverbindung als grünes Pulver. DC(Kieselgel, Essigester/Ligroin 1:1) R_{f}=0.92.

### Beispiel 6

### (4-(Dimethylphosphinylmethyl)-2-methyl-pyrazolo-[1.5a]-imidazol-3-yl)-(4-nitrosophenyl]-amin (11)

13.7 g 3-Amino-2-methyl-4-(dimethylphosphinylmethyl)-pyrazolo-[1.5a]-imidazol Dihydrochlorid werden in 350 ml Methanol gelöst. Die Lösung wird auf ca. 5° C abgekühlt und mit konz. wässriger Natriumkarbonatlösung versetzt, bis auf einem nassen pH-Papier ein pH-Wert von ca. 6 angezeigt wird. Dazu tropft man innerhalb von 30 min eine Lösung von 7.8 g p-Nitrosoanisol in 35 ml Methanol zu. Man rührt die Mischung 4 Stunden bei Raumtemperatur und hält den pH-Wert durch Zugabe von weiterer NaHCO₃-Lösung auf 6.

Das Reaktionsgemisch wird filtriert, mit ca. 150 ml Kieselgel vermischt und zur Trockene eingedampft. Das Kieselgel wird auf eine Kieselgelsäule gepackt und das Produkt durch Eluieren mit Touol/Methanol 2:1 isoliert. Man erhält 10.3 g einer schwarzbraunen Masse, die erneut über Kieselgel mit Methylenchlorid/Methanol 12:1 chromatographiert wird. Man erhält 4.9 g der Titelverbindung mit dem Fp. 204° (unter Zersetzung).

| | |
|---|---|
| Rf (Kieselgel) | Toluol / Methanol 2:1 = 0.3 |
| | CH₂Cl₂/Methanol 12:1 = 0.21 |

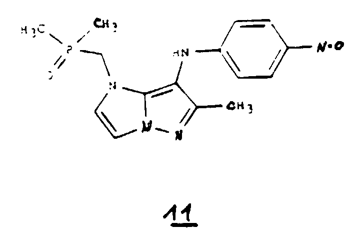

### Herstellung des Ausgangsproduktes

### a) 4-Dimethylphosphinylmethyl-2-methyl-pyrazolo-[1.5a]-imidazol

37 g 2-Methyl-pyrazolo-[1.5a]-imidazol (J. Het. Chem. 10441 (1973) werden in 370 ml trockenem Dimethylformamid gelöst und mit 54.2 g Chlormethyl-dimethylphosphanoxid und 119 g Kaliumcarbonat versetzt. Die Mischung wird 10 Std. bei 115° (Badtemperatur) gerührt. Der Niederschlag wird abgesaugt und das Filtrat eingedampft. Der Rückstand wird an Kieselgel chromatographiert (Laufmittel Essigester/Methanol 2:1). Man erhält insgesamt 36.6 g der Titelverbindung als Mischung von braunen Kristallen und einem braunen Öl. DC (Kieselgel, Essigester/Methanol 2:1).
R_{f} = 0.2

### b) 3-Amino-4-dimethylphosphinyl-2-methyl-pyrazolo-[1.5a]-imidazol x 2 HCl

18 g der oben erhaltenen Verbindung werden in 25 ml konzentrierter Salzsäure und 50 ml Wasser gelöst. Dazu tropft man bei O° eine Lösung von 6.2 g Natriumnitrit in 25 ml Wasser. Nach 30 min. bei O° macht man die Lösung durch Zugabe von Natriumbicarbonatlösung leicht alkalisch und gibt dann portionsweise 21.4 g Natriumdithionit zu. Man rührt das Gemisch noch 30 min und versetzt es mit einer Lösung von 17 g Ditert.-butyldicarbonat in 100 ml Dioxan. Die Reaktionsmischung wird über Nacht bei Raumtemperatur gerührt, das Dioxan abdestilliert und der Rückstand mehrfach mit n-Butanol/Essigester 3:1 extrahiert. Der nach dem Trocknen und Eindampfen der organischen Phase verbleibende Rückstand wird in 320 ml mit HCl gesättigtem Methanol gelöst. Man rührt noch 2 Stunden, kühlt im Eisbad ab und filtriert die ausgefallenen Kristalle ab. Es ergeben sich insgesamt 18.1 g der Titelverbindung. DC (Kieselgel, Toluol/Methanol 3:1) R_{f}=0.1

### Beispiel 7

### (4-Nitrosophenyl)-(4-sulfopropyl-pyrazolo-[1.5a]-imidazol-3-yl)-amin

Analog Beispiel 4 setzt man 3-Amino-4-sulfopropylpyrazolo-[1.5a]-imidazol mit p-Nitrosoanisol in Methanol um. Die Reaktionsmischung wird filtriert und das Filtrat eingedampft. Der Rückstand wird über Kieselgel mit Toluol/Methanol 2:1 chromatographiert. Das Produkt wird dann zur weiteren Reinigung auf eine Säule mit dem Adsorberharz HP 20SS (Fa. Mitsubishi) gegeben und mit einem Stufengradienten von Methanol/Wasser 1:9 bis 2:8 eluiert. Man vereinigt die produkthaltigen Fraktionen, engt ein, nimmt ein wenig Ethanol auf und fällt das Produkt durch Zugabe von Ether aus. Man erhält das Titelprodukt in Form eines braunen Pulvers.
DC (Kieselgel, Ethanol: R_{f}= 0.6,
Isopropanol/Essigsäurebutylester/Wasser 5:3:2:R_{f}=0.48)

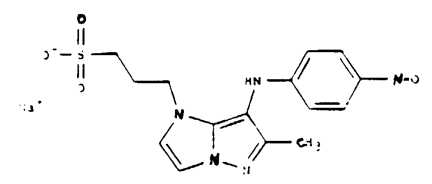

### Beispiel 8

Analog Beispiel 6 erhält man die in der Tabelle 3 aufgeführten Verbindungen.

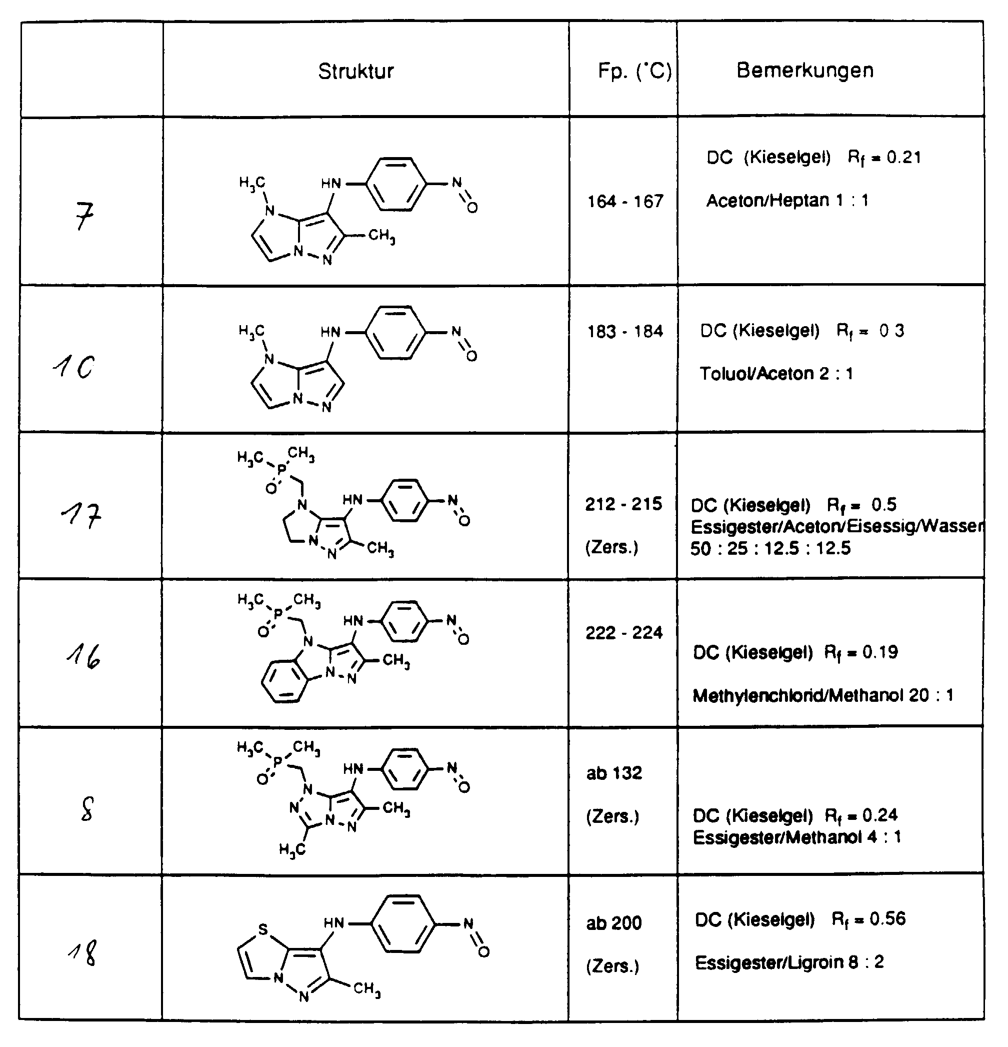

### 1) Herstellung der Ausgangsverbindung zu 17

### 3-Amino-5,6-dihydro-2,4-dimethylphosphinylmethyl-2-methyl-pyrazolo-[1.5a]-imidazol

3.9 g 2-Methyl-pyrazolo-[1.5a]-imidazolin, 2.7 g natriumacetat und 7.4 g p-Methoxy-benzoldiazoniumtetrafluoroborat werden in 40 ml Eisessig gelöst und die Lösung 1 Stunde auf 40-50° erhitzt. Das Reaktionsgemisch wird eingedampft, der Rückstand in NaHCO3-Lösung und Essigester aufgenommen. Man extrahiert mit Essigester, trocknet und dampft ein. Der Rückstand wird durch Chromatographie über Kieselgel gereinigt (Laufmittel: Essigester/Ligroin 1:1-2:1; Essigester; Essigester/Methanol 95:5). Man erhält 4.12 g der entsprechenden 3-Azoverbindung (DC: Kieselgel, Essigester/Methanol 95:5, R_{f}=0.5), die analog Beispiel 6a mit Chlormethyl-dimethylphosphanoxid am Stickstoff des Imidazolinringes alkyliert wird. Die Überführung in die 3-Aminoverbindung erfolgt durch Reduktion der Azogruppe analog Beispiel 6b.

Zur Isolierung und Reinigung wird die rohe Aminoverbindung in wenig Wasser gelöst, mit festem Natriumbicarbonat versetzt und eine Lösung der dreifachen molaren Menge an Di-tert.-butyldicarbonat in Dioxan zugegeben. Man rührt das Gemisch über Nacht, dampft ein, extrahiert zunächst mit Ether, um Nebenprodukte zu entfernen und dann 5 mal mit Methylenchlorid, um das Produkt zu isolieren. Man erhält die kristalline t-Butoxycarbonylverbindung (DC: Essigester/Aceton/Eisessig/Wasser 50:25:12:5, R_{f}=0.5), die zur Abspaltung der tert. Butoxycarbonylgruppe in 50 ml methanolischer Salzsäure gelöst wird. Nach 1 Stunde bei Raumtemperatur engt man auf die Hälfte ein und fällt das Produkt mit Ether aus. Man erhält die Titelverbindung als Hydrochlorid. DC: n-Butanol/Eisessig/Wasser 2:1:1 R_{f}=0.3

### 2) Herstellung der Ausgangsverbindung zu 16

### 3-Amino-4-dimethylphosphinylmethyl-2-methyl-pyrazolo-[1.5a]-benzimidazol

Die Titelverbindung wird analog 5.1. durch Umsetzung von 2-Methyl-pyrazolo-[1.5a]-benzimidazol (J. prakt. Chem. 326, 829 (1984) mit Phenyldiazoniumsalz, N-Alkylierung mit Chlormethyl-dimethylphosphanoxid und Reduktion der Azogruppe mit Zink in Eisessig durchgeführt. Man erhält die Titelverbindung als Tryhydrochlorid-Dihydrat vom Fp. 192 (Zersetzung)
DC: Kieselgel,
Isopropanol/Essigsäurebutylester/Wasser 50:30:20)
R_{f}=0.38

### 3) Herstellung der Ausgangsverbindung zu 8

### 3-Amino-2,6-dimethyl-4-dimethylphosphinyl-pyrazolo-[3.2-c]-s-triazol

### 3.1

6 g 2,6-Dimethyl-pyrazolo-[3.2c]-s-triazol werden in 65 ml Dimethylformamid gelöst und die Lösung mit 3.0 g Chlormethyl-dimethylphosphanoxid und 8 g Kaliumcarbonat versetzt. Man rührt das Gemisch 2 Stunden bei 100°, saugt heiß ab und dampft das Filtrat ein. Der Rückstand wird über Kieselgel chromatographiert/Laufmittel Essigester/Methanol 4:1). Zur Verseifung und Decarboxylierung wird das Produkt 7 Stunden in conc. Salzsäure unter Rückfluß erhitzt. Das Reaktionsgemisch wird eingedampft. Man erhält ein hellbraunes Öl.
DC (Kieselgel Essigester/Methanol 2:1) R_{f}=0.37

### 3.2.

Die Umwandlung des oben erhaltenen Produktes in die 3-Aminoverbindung erfolgt analog dem in Beispiel 3 für das entsprechende 3-Aminopyrazolo-[1.5a]-imidazol beschriebenen Verfahren (3b). Man erhält die Titelverbindung als Hydrochlorid vom Fp. 225° (Zersetzung).
DC (Kieselgel Essigester/Methanol 3:1) R_{f}=0.2

### Beispiel 9

### 3-Nitroso-phenoxazin (19)

2.8 g 2-Amino-2'-hydroxydiphenylether (J. Chem. Soc. 716 (1934) werden in 35 ml Methylenchlorid gelöst und mit 3.4 ml Triethylamin versetzt. Unter Eiskühlung tropft man dazu 3.4 ml Trifluoressigsäureanhydrid, läßt auf Zimmertemperatur kommen und rührt noch 1 Stunde nach. Die Reaktionsmischung wird eingeengt und der Rückstand über Kieselgel mit Essigester/Ligroin 3:7 chromatographiert. Man erhält 3.8 g der entsprechenden N-Trifluoracetylverbindung als zähes Öl.

DC (Kieselgel, Essigester/Ligroin 3:7) R_{f}=0.52

2.2 g Al₂(SO₄)₃ 18 H₂O werden in Wasser gelöst und mit einer Lösung von 1 g der oben erhaltenen Verbindung in 10 ml Dioxan versetzt. Die Reaktionsmischung wird auf 45° erwärmt und auf einmal mit 0.24 g Natriumnitrit versetzt. Nach 1.5 Stunden gibt man nochmals die gleiche Menge Al₂(SO₄)₃, Natriumnitrit und 10 ml Wasser zu, erwärmt noch einige Stunden auf ca. 60°. Danach wird die noch unvollständige Reaktion abgebrochen, das Produkt wird mit Essigester extrahiert. Die getrockente organische Phase wird durch Chromatographie an Kieselgel mit Essigester / Ligroin 1:1 gereinigt. Man erhält 400 mg des orangefarbenen p-Nitrosophenolderivates.
DC (Kieselgel, Essigester/Ligroin 1:1) R_{f}=0.29

320 mg des oben erhaltenen p-Nitrosophenolderivates werden mit 60 mg Natriummethylat in 15 ml Methanol 3 Stunden unter Rückfluß gekocht. Unter diesen Bedingungen wird die Trifluoracetylgruppe abgespalten und das Zwischenprodukt cyclisiert zu der gewünschten Oxazinverbindung. Zur Reinigung wird der Rückstand der eingedampften Reaktionsmischung über Kieselgel mit Essigester/Ligroin 3:7 chromatographiert. Man erhält 50 mg der Titelverbindung als orangefarbene Substanz, die sich oberhalb von 200° zersetzt.
DC (Kieselgel, Essigester/Ligroin 3:7) R_{f}=0.34

### Beispiel 10

### (2-Methyl-pyrazolo-[1.5a]-pyridin-3-yl)-(4-nitroso-2-sulfonropyl-phenyl)-amin (20)

10 g 2-(3'-Hydroxypropyl)-phenol und 37 ml einer 20 proz. Lösung von Tetramethylammoniumhydroxid in Methanol werden in 80 ml Dimethylformamid gelöst und bei Raumtemperatur tropfenweise mit 8.5 ml Benzylbromid versetzt. Man rührt das Gemisch noch 2 Stunden bei Raumtemperatur, dampfe ein und verteilt den Rückstand zwischen Essigester und schwach angesäuertem Wasser.
Die organische Phase wird abgetrennt und getrocknet. Man erhält 16.0 g O-Benzyl-2-(3'hydroxypropyl)-phenol in Form eines schwach gelben Öls.
DC (Kieselgel Essigester/Ligroin 65:35) R_{f}=0.54

Analog Organic Synthesis Coll. Vol. V, S. 249 wird die oben erhaltene Verbindung mittels des TriphenylphosphinBrom-Adduktes in das (3-Brompropyl)-phenol umgewandelt. Die Verbindung wird durch Chromatographie an Kieselgel mit Ligroin/Ethanol 9:1 gereinigt. Das Produkt wird in Wasser / Methanol 20:7 gelöst mit der 2.5 fachen molaren Menge Natriumsulfit 7H2O und ca. 90 mol % Tetrabutylammoniumbromid versetzt. Das Reaktionsgemisch wird 2 Stunden unter Rückfluß erhitzt, eingedampft und mit Wasser versetzt. Das Produkt wird mit Essigester extrahiert. Man erhält die 2-Benzyloxy-phenyl-propansulfonsäure als farbloses Öl in Form des Tetrabutylammoniumsalzes.
DC (Kieselgel, Toluol/Methanol/Eisessig 8:15:5) R_{f}=0.29

Zur Abspaltung des Benzylrestes wird die Substanz in methanol über Pd/C hydriert. Das Filtrat des Hydrierungsansatzes wird eingeengt und zur Freisetzung der Sulfonsäure über einen stark sauren Ionenaustauscher (DOWEX 50 Wx4) mit Wasser filtriert. Durch Einengen der entsprechenden Fraktionen erhält man die freie Sulfonsäure als eine teilweise kristalline Masse.

0,5 g der oben erhaltenen Sulfonsäure werden in 17 ml Wasser gelöst und mit 160 mg Natriumnitrit versetzt. Die Reaktionsmischung wird 4 Stunden bei Raumtemperatur gerührt, eingeengt, in wenig Methanol gelöst und das Produkt mit Ether ausgefällt. Man erhält 370 mg 2-Hydroxy-5-nitroso-propansulfonsäure-Natriumsalz.
DC (Kieselgel Essigester/Aceton/Eisessig/Wasser 50:25:12:5:12) R_{f}=0.54

Das oben erhaltene Nitrosophenol wird in Methanol 7 Stunden unter Rückfluß erhitzt. Es stellt sich ein Gleichgewicht ein zwischen dem Ausgangsprodukt und dem sprechenden Nitrosoanisol.

Die Reaktionsmischung wird eingeengt und ohne weitere Reinigung analog Beispiel 3 mit 3-Amino-2-methyl-pyrazolo-[1.5a]-pyridin umgesetzt. Man erhält die Titelverbindung als gelbgrüne Substanz vom Fp.>170 (Zersetzung). DC (Kieselgel Essigester/Methanol 75:25) Rf=0.38

## Patentansprüche

1. Verfahren zur kolorimetrischen Bestimmung von NAD(P)H oder eines Analyten, der ein Substrat eines unter Bildung von NAD(P)H reagierenden Enzyms ist, mittels Oxidation mit einem NAD(P)H-oxidierenden Enzym in Anwesenheit eines direkten chromogenen Elektronenakzeptors und Bestimmung des reduzierten Elektronenakzeptors als Maß für die Menge an NAD(P)H, dadurch gekennzeichnet, daß NAD(P)H mit dem Enzym Benzylalkoholdehydrogenase in Anwesenheit einer Nitrosoanilinverbindung der Formel I 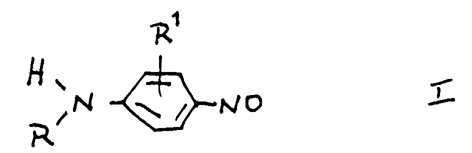 wobei
R1 = Wasserstoff Halogen, Hydroxy, C₁-C₆ Akoxy,C₆-C₁₀ Aryloxy, C₁-C₆ Alkylthio oder C₆-C₁₀ Arylthio, C₁-C₆ Alkyl oder C₂-C₅ Alkylen gegebenenfalls substituiert durch Carboxy, P0₃H₂, Di-(C₁-C₆)alkylphosphinyl oder SO₃H,Amino, gegebenenfalls ein- oder zweimal substituiert durch C₁-C₆ Alkyl, das wiederum gegebenenfalls durch Hydroxy, P0₃H₂, Di-(C₁-C₆) alkylphosphinyl, S0₃H oder Carboxy substituiert sein kann, bedeutet und
R = einen durch Elektronenkonjugation mit einem Chinondiimin-System farberzeugenden Rest bedeutet,
als Elektronenakzeptor umgesetzt wird, dabei die Nitrosoanilinverbindung zu einem farbigen Chinondiimin enzymatisch reduziert wird und die Farbe des Chinondiimins als Maß für die Menge an NAD(P)H gemessen wird.

2. Verfahren gemäß Anspruch 1, dadurch gekennzeichnet, daß als farberzeugender Rest ein Rest der allgemeinen Formel III eingesetzt wird. 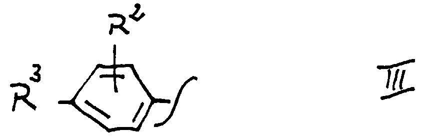 in der
R² Wasserstoff Hydroxy, Halogen, C₁-C₆ Alkoxy, C₁-C₆ Alkylthio, C₆-C₁₀ Aryloxy oder C₆-C₁₀ Arylthio, C₁-C₆ Alkyl oder C₂-C₅ Alkylen, gegebenenfalls substituiert durch Carboxy, P0₃H₂ oder SO₃H, Amino, gegebenenfalls ein- oder zweimal substituiert durch C₁-C₆ Alkyl, das gegebenenfalls wiederum durch Hydroxy, P0₃H₂, SO₃H oder Carboxy substituiert sein kann, bedeutet oder mit dem Rest R¹ eine -CH₂-CH₂-, -CH₂-0-, -CR¹² = CR¹³ -,-0-, -S- oder -NH-Brücke bilden kann, wobei -NH- gegebenenfalls durch einen C₁-C₆ Alkylrest substituiert sein kann, und R¹² und R¹³ Carboxy oder C₁-C₆ Alkyl bedeutet, das gegebenenfalls durch Hydroxy, Carboxy, P0₃H₂ oder SO₃H substituiert sein kann.
R³ eine Hydroxygruppe, C₁-C₆ Alkyl-, C₁-C₆ Alkoxy-, C₆-C₁₀ Aryl-, C₆-C₁₀ Aryloxy-, C₆-C₁₀ Arylthio- oder C₁-C₆ Alkylthiogruppe bedeutet, wobei der Alkylrest gegebenenfalls seinerseits durch eine Hydroxygruppe, eine C₁-C₆ Alkoxygruppe, eine gegebenenfalls ein- oder mehrfach durch C₁-C₆ Alkyl substituierte Aminogruppe, P0₃H₂, SO₃H oder CO₂H als solche oder in Salzform als Ammonium-, Alkali- oder Erdalkalisalze substituiert ist,
oder eine Aminogruppe NR⁴R⁵ bedeutet,
in der R⁴ und R⁵ gleich oder verschieden sein können, und Wasserstoff eine C₁-C₆ Alkylgruppe, die ihrerseits durch eine Hydroxy-, C₁-C₆ Alkoxy-, Hydroxy-(C₁-C₆)alkoxy-, eine gegebenenfalls hydroxysubstituierte Poly-(C₁-C₆)alkoxygruppe, P0₃H₂, S0₃H, COOH als solche oder in Salzform oder durch eine gegebenenfalls ein- oder mehrfach durch C₁-C₆ Alkyl substituierte Aminogruppe substituiert sein kann, bedeutet, oder
in der R⁴ und R⁵ einen C₂-C₅ Alkylenrest darstellen können, der gegebenenfalls durch Sauerstoff, Schwefel oder Stickstoff unterbrochen ist, wobei Stickstoff durch einen C₁-C₆ Alkyl-, Hydroxy-(C₁-C₆)alkyl-, Hydroxy-(C₁-C₆)alkoxy-(C₁-C₆)alkyl-, C₁-C₆ Alkoxyhydroxy-(C₁-C₆)alkyl-, C₁-C₆ Alkoxycarbonyl-(C₁-C₆)alkyl-, Dioxanylyl-(C₁-C₆)alkyl- oder Poly-(C₁-C₆)alkoxy-(C₁-C₆)alkylrest substituiert ist, der seinerseits jeweils gegebenenfalls im Alkylteil durch einen Hydroxyrest substituiert sein kann, oder wenn R² in ortho-Stellung zu NR⁴R⁵ steht, R⁴ oder R⁵ auch zusammen mit R² einen C₂-C₅ Alkylenrest darstellen kann.

3. Verfahren gemäß Anspruch 2, dadurch gekennzeichnet, daß R einen N,N-Dihydroxyethylanilin-, -N,N-Dimethylanilin- oder N,N-Diethylanilin-Rest, darstellt.

4. Verfahren gemäß Anspruch 1, dadurch gekennzeichnet, daß als farberzeugender Rest ein Rest der allgemeinen Formel IV eingesetzt wird, wobei 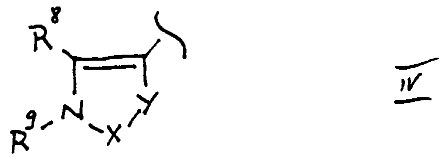
X-Y NR⁶-CO oder N=CR⁷ darstellt, wobei
R⁶ H, C₁-C₆ Alkyl gegebenenfalls substituiert durch Hydroxy, Carboxy, SO₃H, P0₃H₂, Di-(C₁-C₆)alkylphosphinyl,
R⁷ H, C₁-C₆ Alkyl, C₂-C₅ Alkenyl, C₁-C₆ Alkoxy, C₁-C₆ Alkylthio, C₆-C₁₀ Aryl, C₆-C₁₀ Ar-(C₁-C₆)alkyl, gegebenenfalls jeweils substituiert durch Hydroxy, Di-(C₁-C₆)alkylphosphinyl, Carboxy, SO₃H, P0₃H₂ ein Salz eines dieser Säurereste oder/und C₁-C₆ Alkoxycarbonyl, oder
Amino, das gegebenenfalls durch einen oder zwei gegebenenfalls einen oder mehrere Hydroxy-, Carboxy oder/und C₁-C₆ Alkoxy-carbonylreste tragende C₁-C₆ Alkylreste substituiert ist, wobei wenn Amino durch 2 C₁-C₆ Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann oder Amino, gegebenenfalls durch ein oder zwei Acylgruppen, C₁-C₆ Alkoxy- oder/und C₆-C₁₀ Ar-(C₁-C₆)alkoxycarbonylgruppen, H₂N-CO, C₁-C₆ Alkyl-, C₆-C₁₀ Ar-(C₁-C₆)alkyl oder/und C₆-C₁₀ Arylcarbamoylgruppen substituiert ist; oder Wasserstoff, Carboxy, C₁-C₆ Alkoxycarbonyl, Carboxamido oder Halogen ist und
R⁸ C₁-C₆ Alkyl, C₁-C₆ Thioalkyl oder C₆-C₁₀ Ar-(C₁-C₆)alkyl, gegebenenfalls substituiert durch Hydroxy, Carboxy, SO₃H oder P0₃H₂ oder Amino, das gegebenenfalls durch eine oder zwei C₁-C₆ Alkylgruppen substituiert ist, die wiederum durch Hydroxy, Carboxy, SO₃H, Di-(C₁-C₆)alkylphosphinyl oder P0₃H₂ substituiert sein können, wobei mindestens R⁷ und/oder R⁸ eine Aminogruppe darstellt, und
R⁹ eine C₁-C₆ Alkyl oder C₆-C₁₀ Ar-(C₁-C₆)alkylgruppe, die gegebenenfalls durch Hydroxy, Carboxy, SO₃H oder P0₃H₂ substituiert sein kann
oder wobei R⁸ und R⁹ zusammen eine gesättigte oder ungesättigte Kette aus 3 oder 4 Gliedern aus Stickstoffatomen oder aus Kohlenstoffatomen und gegebenenfalls einem oder mehreren Stickstoff- oder Schwefelatomen bilden können, wobei Kohlenstoffatome gegebenenfalls substituiert sind durch C₁-C₆ Alkyl, C₁-C₆ Alkoxy, C₁-C₆ Alkylthio, Hydroxy, C₆-C₁₀ Ar-(C₁-C₆)alkyl, C₆-C₁₀ Aryl, Carboxy, Carboxamido, C₁-C₆ Alkoxycarbonyl, Cyano, Halogen, Amino, das gegebenenfalls durch einen oder zwei, gegebenenfalls einen oder mehrere Hydroxy-, Carboxy oder/und C₁-C₆ Alkoxycarbonylreste tragende C₁-C₆ Alkylreste substituiert ist, und wobei Stickstoffatome, die nicht über eine Doppelbindung gebunden sind, durch Wasserstoff, C₁-C₆ Alkyl gegebenenfalls durch Hydroxy, SO₃H, P0₃H₂, COOH oder Di-(C₁-C6)alkylphosphinyl substituiert, oder durch C₆-C₁₀ Ar-(C₁-C₆)alkyl substituiert sind oder zwei benachbarte Kettensubstituenten gegebenenfalls eine C₃-C₄ Alkylengruppe bilden, die ihrerseits gegebenenfalls mit C₆-C₁₀ Aryl substituiert oder anelliert ist
sowie gegebenenfalls entsprechende tautomere Formen und deren Salze.

5. Verfahren gemäß Anspruch 4, dadurch gekennzeichnet, daß R einen Rest der Formeln V bis XIV darstellt, 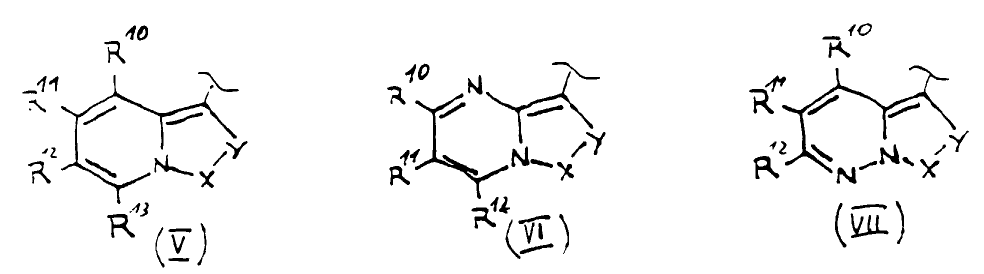 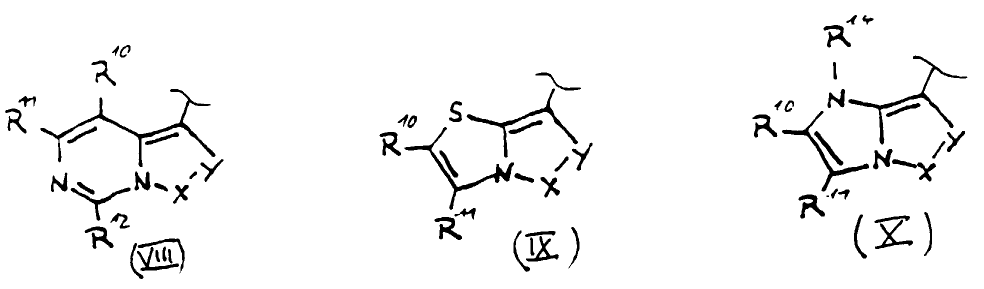 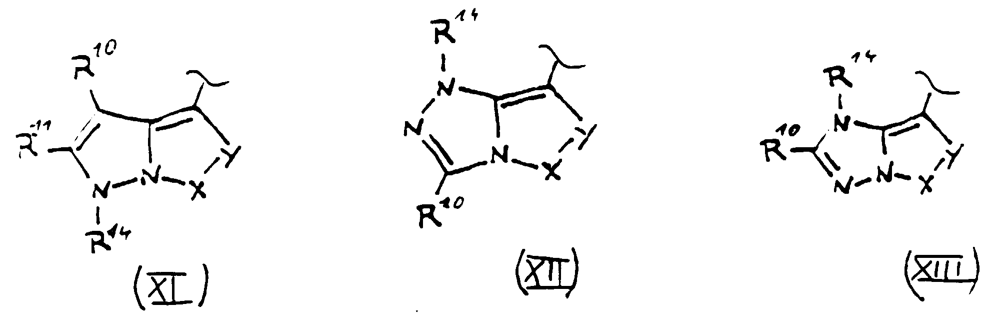 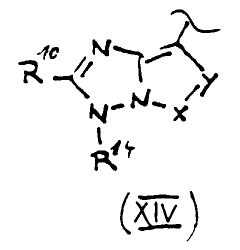 in dem R¹⁰, R¹¹, R¹² und R¹³, die gleich oder verschieden sind, für Wasserstoff, Hydroxy, C₁-C₆ Alkyl, C₁-C₆ Alkoxy, C₁-C₆ Alkylthio, C₆-C₁₀ Ar-(C₁-C₆)alkyl, C₆-C₁₀ Aryl, Carboxy, C₁-C₆ Alkoxycarbonyl, Carboxamido, Cyano, Amino, das gegebenenfalls durch ein oder zwei gegebenenfalls einen oder mehrere Hydroxy-, Carboxy- oder/und C₁-C₆ Alkoxycarbonylreste tragende C₁-C₆ Alkylreste substituiert ist oder Halogen stehen, wobei zwei benachbarte Reste gegebenenfalls eine C₃-C₄ Alkylengruppe bilden, die ihrerseits gegebenenfalls mit C₆-C₁₀ Aryl substituiert oder anelliert ist und R¹⁴ C₁-C₆ Alkyl oder C₆-C₁₀ Ar-(C₁-C₆)alkyl darstellt, das gegebenenfalls durch Hydroxy, Carboxy, SO₃H, P0₃H₂ oder Di-(C₁-C₆)alkylphosphinyl substituiert ist.

6. Nitrosoanilinverbindung der Formel I, 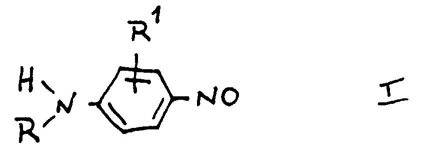 in der
R¹ Wasserstoff, Halogen, Hydroxy, C₁-C₆ Alkoxy, C₆-C₁₀ Aryloxy, C₁-C₆ Alkylthio oder C₆-C₁₀ Arylthio, C₁-C₆ Alkyl oder C₂-C₅ Alkylen gegebenenfalls substituiert durch Carboxy, P0₃H₂, Di-(C₁-C₆)alkylphosphinyl oder SO₃H, Amino, gegebenenfalls ein- oder zweimal substituiert durch C₁-C₆ Alkyl, das wiederum gegebenenfalls durch Hydroxy, P0₃H₂, SO₃H oder Carboxy substituiert sein kann, bedeutet und
R einen durch Elektronenkonjugation mit einem Chinondiimin-System farberzeugenden Rest bedeutet.

7. Nitrosoanilinverbindung gemäß Anspruch 6, dadurch gekennzeichnet, daß R einen Rest der allgemeinen Formel III darstellt, 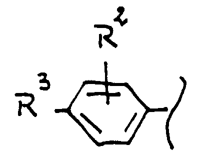 in der
R² Wasserstoff, Hydroxy, Halogen, C₁-C₆ Alkoxy oder C₁-C₆ Alkylthio, C₆-C₁₀ Aryloxy oder C₆-C₁₀ Arylthio, C₁-C₆ Alkyl oder C₂-C₅ Alkylen, gegebenenfalls substituiert durch Carboxy, P0₃H₂ oder SO₃H, Amino, gegebenenfalls ein- oder zweimal substituiert durch C₁-C₆ Alkyl, das gegebenenfalls wiederum durch Hydroxy, P0₃H₂, SO₃H oder Carboxy substituiert sein kann, bedeutet oder mit dem Rest R¹ eine -CH₂-CH₂-, -CH₂- 0-, -CR¹² = CR¹³-, -0-, -S-, oder -NH-Brücke bilden kann, wobei -NH- gegebenenfalls durch C₁-C₆ Alkyl substituiert sein kann und R¹² und R¹³ Carboxy oder C₁-C₆ Alkyl bedeutet, das gegebenenfalls durch Hydroxy, Carboxy, P0₃H₂ oder SO₃H substituiert sein kann und
R³ eine Hydroxygruppe, C₁-C₆ Alkyl-, C₁-C₆ Alkoxy-, C₆-C₁₀ Aryl-, C₆-C₁₀ Aryloxy-, C₆-C₁₀ Arylthio oder C₁-C₆ Alkylthiogruppe bedeutet, wobei der Alkylrest gegebenenfalls seinerseits durch eine Hydroxygruppe, eine C₁-C₆ Alkoxygruppe, eine gegebenenfalls ein- oder mehrfach durch C₁-C₆ Alkyl substituierte Aminogruppe, P0₃H₂, SO₃H oder CO₂H als solche in Salzform als Ammonium-, Alkali- oder Erdalkalisalze substituiert ist,
oder eine Aminogruppe NR⁴R⁵ bedeutet,
in der R⁴ und R⁵ gleich oder verschieden sein können, und Wasserstoff, eine C₁-C₆ Alkylgruppe, die ihrerseits durch eine Hydroxy-, C₁-C₆ Alkoxy-, Hydroxy-(C₁-C₆)alkoxy-, eine gegebenenfalls hydroxysubstituierte Poly-(C₁-C₆)alkoxygruppe, P0₃H₂, SO₃H, COOH als solche oder in Salzform oder durch eine gegebenenfalls ein- oder mehrfach durch C₁-C₆ Alkyl substituierte Aminogruppe substituiert sein kann, bedeutet, oder
in der R⁴ und R⁵ einen C₂-C₅ Alkylenrest darstellen können, der gegebenenfalls durch Sauerstoff, Schwefel oder Stickstoffunterbrochen ist, wobei Stickstoffdurch einen C₁-C₆ Alkyl-, Hydroxy-(C₁-C₆)alkyl-, Hydroxy-(C₁-C₆)alkoxy-(C₁-C₆)alkyl-, C₁-C₆ Alkoxyhydroxy-(C₁-C₆)alkyl-, C₁-C₆ Alkoxycarbonyl-(C₁-C₆)alkyl-, Dioxanylyl-(C₁-C₆)alkyl- oder Poly-(C₁-C₆)alkoxy-(C₁-C₆)alkylrest substituiert ist, der seinerseits jeweils gegebenenfalls im Alkylteil durch einen Hydroxyrest substituiert sein kann, oder wenn R² in ortho-Stellung zu NR⁴R⁵ steht, R⁴ oder R⁵ auch zusammen mit R² einen C₂-C₅ Alkylenrest darstellen kann.

8. Nitrosoanilinverbindung gemäß Anspruch 6, dadurch gekennzeichnet, daß R einen Rest der allgemeinen Formel IV darstellt, 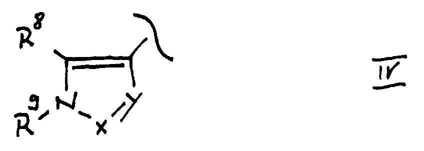 wobei
X-Y NR⁶-CO oder N=CR⁷ darstellt, wobei
R⁶ H, C₁-C₆ Alkyl gegebenenfalls substituiert durch Hydroxy, Carboxy, SO₃H, P0₃H₂, Di-(C₁-C₆)alkylphosphinyl
R⁷ H, C₁-C₆ Alkyl, C₂-C₅ Alkenyl, C₁-C₆ Alkoxy, C₁-C₆ Alkylthio, C₆-C₁₀ Aryl, C₆-C₁₀ Ar-(C₁-C₆)alkyl, gegebenenfalls jeweils substituiert durch Hydroxy, Di-(C₁-C₆)alkylphosphinyl, Carboxy, SO₃H, P0₃H, ein Salz eines dieser Säurereste oder/und C₁-C₆ Alkoxycarbonyl, oder
Amino, das gegebenenfalls durch einen oder zwei gegebenenfalls eine oder mehrere Hydroxy-, Carboxy oder/und C₁-C₆ Alkoxy-carbonylreste tragende C₁-C₆ Alkylreste substituiert ist wobei, wenn Amino durch 2 C₁-C₆ Alkylreste substituiert ist, diese Reste auch zu einem Ring geschlossen sein können, der außer durch das N-Atom der Aminogruppe gegebenenfalls auch durch Sauerstoff, Schwefel oder ein weiteres Stickstoffatom unterbrochen sein kann oder Amino gegebenenfalls durch ein oder zwei Acylgruppen, C₁-C₆ Alkoxy- oder/und C₆-C₁₀ Ar-(C₁-C₆)alkoxycarbonylgruppen, H₂N-C0, C₁-C₆ Alkyl-, C₆-C₁₀ Ar-(C₁-C₆)alkyl oder/und C₆-C₁₀ Arylcarbamoylgruppen substituiert ist; oder Wasserstoff, Carboxy, C₁-C₆ Alkoxycarbonyl, Carboxamido oder Halogen ist und
R⁸ C₁-C₆ Alkyl, C₁-C₆ Thioalkyl oder C₆-C₁₀ Ar-(C₁-C₆)alkyl, gegebenenfalls substituiert durch Hydroxy, Carboxy, SO₃H oder P0₃H₂ oder Amino, das gegebenenfalls durch eine oder zwei C₁-C₆ Alkylgruppen substituiert ist, die wiederum durch Hydroxy, Carboxy, SO₃H, Di-(C₁-C₆)alkylphosphinyl oder P0₃H₂ substituiert sein können,
wobei mindestens R⁷ und/oder R⁸ eine Aminogruppe darstellt, und
R⁹ eine C₁-C₆ Alkyl oder C₆-C₁₀ Ar-(C₁-C₆)alkylgruppe, die gegebenenfalls durch Hydroxy, Carboxy, SO₃H oder P0₃H₂ substituiert sein kann
oder wobei R⁸ und R⁹ zusammen
eine gesättigte oder ungesättigte Kette mit 3 oder 4 Gliedern aus Stickstoffatomen oder aus Kohlenstoffatomen und gegebenenfalls einem oder mehreren Stickstoff- oder Schwefelatomen bilden können, wobei Kohlenstoffatome gegebenenfalls substituiert sind durch C₁-C₆ Alkyl, C₁-C₆ Alkoxy, C₁-C₆ Alkylthio, Hydroxy, C₆-C₁₀ Ar-(C₁-C₆)alkyl, C₁-C₆ Aryl, Carboxy, Carboxamido, C₁-C₆ Alkoxycarbonyl, Cyano, Halogen, Amino, das gegebenenfalls durch einen oder zwei, gegebenenfalls einen oder mehrere Hydroxy-,Carboxy oder/und C₁-C₆ Alkoxycarbonylreste tragende C₁-C₆ Alkylreste substituiert ist und wobei Stickstoffatome, die nicht über eine Doppelbindung gebunden sind, durch Wasserstoff, C₁-C₆ Alkyl gegebenenfalls durch Hydroxy, SO₃H, P0₃H₂, COOH oder Di-(C₁-C₆)alkylphosphinyl substituiert, oder durch C₆-C₁₀ Ar-(C₁-C₆)alkyl substituiert sind oder zwei benachbarte Kettensubstituenten gegebenenfalls eine C₃-C₄ Alkylengruppe bilden, die ihrerseits gegebenenfalls mit C₆-C₁₀ Aryl substituiert oder anelliert ist sowie gegebenenfalls entsprechende tautomere Formen und deren Salze

9. Mittel zur kolorimetrischen Bestimmung von NAD(P)H, bestehend aus einem NAD(P)H-oxidierenden Enzym und einem direkten chromogenen Elektronenakzeptor, dadurch gekennzeichnet, daß es Benzylalkoholdehydrogenase und eine Nitrosoanilinverbindung gemäß einem der Ansprüche 6 -8 enthält.

10. Mittel zur kolorimetrischen Bestimmung von Benzylalkoholdehydrogenase, enthaltend NAD(P)H und einen direkten chromogenen Elektronenakzeptor, dadurch gekennzeichnet, daß der Elektronenakzeptor eine Nitrosoanilinverbindung gemäß einem der Ansprüche 6 - 8 ist.

11. Mittel zur kolorimetrischen Bestimmung einer NAD(P)⁺-abhängigen Dehydrogenase, enthaltend ein Substrat der zu bestimmenden Dehydrogenase, NAD(P)⁺, eine NAD(P)H-umsetzende Dehydrogenase und einen direkten chromogenen Elektronenakzeptor, dadurch gekennzeichnet, daß die NAD(P)H-umsetzende Dehydrogenase Benzylalkoholdehydrogenase und der Elektronenakzeptor eine Nitrosoanilinverbindung gemäß einem der Ansprüche 6 - 8 ist.

12. Mittel zur kolorimetrischen Bestimmung eines Analyten, der ein Substrat eines unter Bildung von NAD(P)H-reagierenden Enzyms ist, enthaltend eine analyt-spezifische NAD(P)-abhängige Dehydrogenase, NAD(P), ein NAD(P)H-oxidierendes Enzym und einen direkten chromogenen Elektronenakzeptator, dadurch gekennzeichnet, daß es als NAD(P)H-oxidierendes Enzym Benzylalkoholdehydrogenase und als direkten chromogenen Elektronenakzeptor eine Nitrosoanilinverbindung gemäß einem der Ansprüche 6 -8 enthält.

13. Verfahren zur Herstellung der Nitrosoanilinverbindung der Formel I gemäß Anspruch 6, dadurch gekennzeichnet, daß ein p-Nitrosophenylether der Formel XVII mit einer Aminoverbindung der Formel XVIII R-NH2 umgesetzt wird.

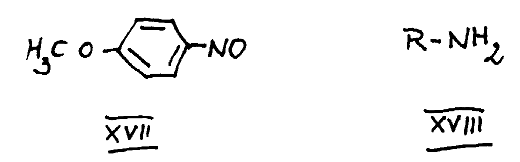

14. Verwendung von Nitrosoanilinverbindungen gemäß einem der Ansprüche 6 - 8 zur kolorimetrischen enzymatischen Bestimmung eines Analyten.

## Claims

1. Method for the colorimetric determination of NAD(P)H or of an analyte which is a substrate of an enzyme which reacts to form NAD(P)H, by means of oxidation with a NAD(P)H-oxidizing enzyme in the presence of a direct chromogenic electron acceptor and determination of the reduced electron acceptor as a measure of the amount of NAD(P)H, wherein NAD(P)H is reacted with the enzyme benzyl alcohol dehydrogenase in the presence of a nitrosoaniline compound of the general formula I 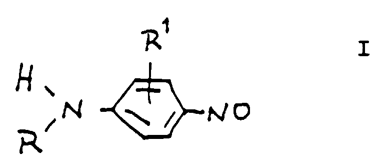 in which
R¹ = denotes hydrogen, halogen, hydroxy, C₁-C₆ alkoxy, C₆-C₁₀ aryloxy, C₁-C₆ alkylthio or C₆-C₁₀ arylthio, C₁-C₆ alkyl or C₂-C₅ alkylene optionally substituted by carboxy, PO₃H₂, di-(C₁-C₆)alkylphosphinyl or SO₃H, amino substituted if desired once or twice by C₁-C₆ alkyl which in turn can optionally be substituted by hydroxy, PO₃H_{2,} di-(C₁-C₆) alkylphosphinyl, SO₃H or carboxy and
R = denotes a residue which forms a colour by electron conjugation with a quinone diimiine system,
as an electron acceptor, the nitrosoaniline compound being enzymatically reduced to a coloured quinone diimiine in this process and the colour of the quinone diimiine is measured as a measure of the amount of NAD(P)H.

2. Method as claimed in claim 1, wherein a residue of the general formula III is used as the colour-forming residue. 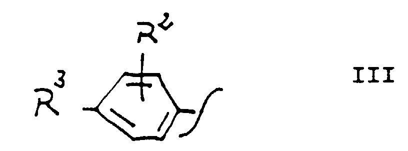 in which
R² denotes hydrogen, hydroxy, halogen, C₁-C₆ alkoxy, C₁-C₆ alkylthio, C₆-C₁₀ aryloxy or C₆-C₁₀ arylthio, C₁-C₆ alkyl or C₂-C₅ alkylene, substituted if desired by carboxy, PO₃H₂ or SO₃H, amino which is optionally substituted once or twice by C₁-C₆ alkyl which in turn can be substituted if desired by hydroxy, PO₃H₂, SO₃H or carboxy or can form a -CH₂-CH₂-, -CH₂-O-, -CR¹²=CR¹³-, -O-, -S- or -NH- bridge with the residue R¹ in which NH can be substituted if desired by a C₁-C₆ alkyl residue and R¹² and R¹³ denote carboxy or C₁-C₆ alkyl which can be substituted if desired by hydroxy, carboxy, PO₃H₂ or SO₃H.
R³ denotes a hydroxy group, C₁-C₆ alkyl, C₁-C₆ alkoxy, C₆-C₁₀ aryl, C₆-C₁₀ aryloxy, C₆-C₁₀ arylthio or C₁-C₆ alkylthio group wherein the alkyl residue is in turn substituted if desired by a hydroxy group, a C₁-C₆ alkoxy group, an amino group substituted if desired once or several times by C₁-C₆ alkyl, PO₃H₂, SO₃H or CO₂H as such or in a salt form as ammonium, alkali or alkaline earth salts, or denotes an amino group NR⁴R⁵,
in which R⁴ and R⁵ can be the same or different and denote hydrogen, a C₁-C₆ alkyl group which in turn can be substituted by a hydroxy, C₁-C₆ alkoxy, hydroxy-(C₁-C₆)alkoxy, a poly-(C₁-C₆)alkoxy group which is substituted if desired by hydroxy, PO₃H₂, SO₃H, COOH as such or in a salt form or by an amino group which is substituted if desired once or several times by C₁-C₆ alkyl, or
in which R⁴ and R⁵ can represent a C₂-C₅ alkylene residue which is interrupted if desired by oxygen, sulphur or nitrogen, wherein nitrogen is substituted by a C₁-C₆ alkyl, hydroxy-(C₁-C₆)alkyl, hydroxy-(C₁-C₆)-alkoxy-(C₁₋C₆)alkyl, C₁-C₆ alkoxyhydroxy-(C₁-C₆)alkyl, C₁-C₆ alkoxycarbonyl-(C₁-C₆)alkyl, dioxanylyl-(C₁-C₆)alkyl or poly-(C₁-C₆) alkoxy-(C₁-C₆)alkyl residue, each of which in turn can be substituted if desired in the alkyl moiety by a hydroxy residue or if R² is in the ortho position in relation of NR⁴R⁵, R⁴ or R⁵ together with R² can also represent a C₂-C₅ alkylene residue.

3. Method as claimed in claim 2, wherein R represents a N,N-dihydroxyethylaniline, N,N-dimethylaniline or N,N-diethylaniline residue.

4. Method as claimed in claim 1, wherein a residue of the general formula IV is used as a colour-forming residue, 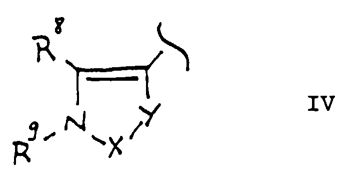 wherein
X-Y represents NR⁶-CO or N=CR⁷ in which
R⁶ represents H, C₁-C₆ alkyl substituted if desired by hydroxy, carboxy, SO₃H, PO₃H₂, di-(C₁-C₆)alkylphosphinyl,
R⁷ represents H, C₁-C₆ alkyl, C₂-C₅ alkenyl, C₁-C₆ alkoxy, C₁-C₆ alkylthio, C₆-C₁₀ aryl, C₆-C₁₀ ar-(C₁-C₆)alkyl, each of which is optionally substituted by hydroxy, di-(C₁-C₆)alkylphosphinyl, carboxy, SO₃H, PO₃H₂, a salt of one of these acid residues or/and C₁-C₆ alkoxycarbonyl, or
amino which is optionally substituted by one or two C₁-C₆ alkyl residues carrying if
desired one or several hydroxy, carboxy or/and C₁-C₆ alkoxycarbonyl residues, wherein if amino is substituted by 2 C₁-C₆ alkyl residues these residues can also be linked to form a ring which, apart from the N atom of the amino group, can also be interrupted by oxygen, sulphur or a further nitrogen atom or amino which is optionally substituted by one or two acyl groups, C₁-C₆ alkoxy or/and C₆-C₁₀ ar-(C₁-C₆)alkoxycarbonyl groups, H₂N-CO, C₁-C₆ alkyl, C₆-C₁₀ ar-(C₁-C₆)alkyl or/and C₆-C₁₀ arylcarbamoyl groups; or is hydrogen, carboxy, C₁-C₆ alkoxycarbonyl, carboxamido or halogen and
R⁸ denotes C₁-C₆ alkyl, C₁-C₆ thioalkyl or C₆-C₁₀ ar-(C₁-C₆)alkyl optionally substituted by hydroxy, carboxy, SO₃H or PO₃H₂ or amino which is substituted if desired by one or two C₁-C₆ alkyl groups which in turn can be substituted by hydroxy, carboxy, SO₃H, di-(C₁-C₆)alkylphosphinyl or PO₃H₂, wherein at least R⁷ and/or R⁸ represents an amino group, and
R⁹ represents a C₁-C₆ alkyl or C₆-C₁₀ ar-(C₁-C₆)alkyl group which can optionally be substituted by hydroxy, carboxy, SO₃H or PO₃H₂
or wherein R⁸ and R⁹ together represent a saturated or unsaturated chain with 3 or 4 members composed of nitrogen atoms or carbon atoms and if desired of one or several nitrogen or sulphur atoms, wherein the carbon atoms are optionally substituted by C₁-C₆
alkyl, C₁-C₆ alkoxy, C₁-C₆ alkylthio, hydroxy, C₆-C₁₀ ar-(C₁-C₆)alkyl, C₆-C₁₀ aryl, carboxy, carboxamido, C₁-C₆ alkoxycarbonyl, cyano, halogen, amino which is substituted if desired by one or two C₁-C₆ alkyl residues optionally carrying one or several hydroxy, carboxy or/and C₁-C₆ alkoxycarbonyl residues and wherein nitrogen atoms which are not bound via a double bond are substituted by hydrogen, C₁-C₆ alkyl optionally substituted by hydroxy, SO₃H, PO₃H₂, COOH or di-(C₁-C₆)alkylphosphinyl, or by C₆-C₁₀ ar-(C₁-C₆)alkyl or two adjacent chain substituents if desired form a C₃-C₄ alkylene group which in turn is substituted if desired by C₆-C₁₀ aryl or is anellated,
as well as optionally appropriate tautomeric forms and their salts.

5. Method as claimed in claim 4, wherein R represents a residue of formulae V to XIV, 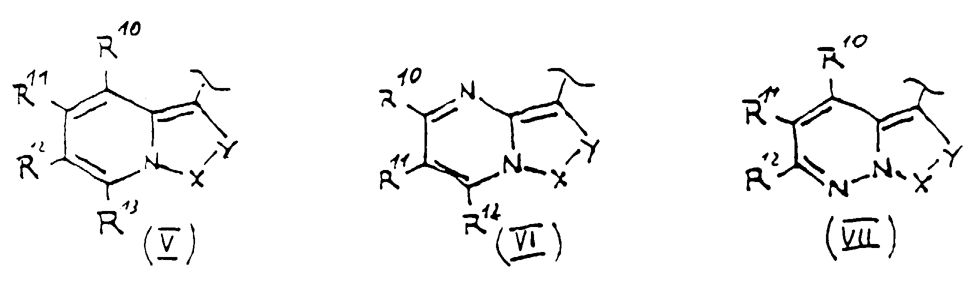 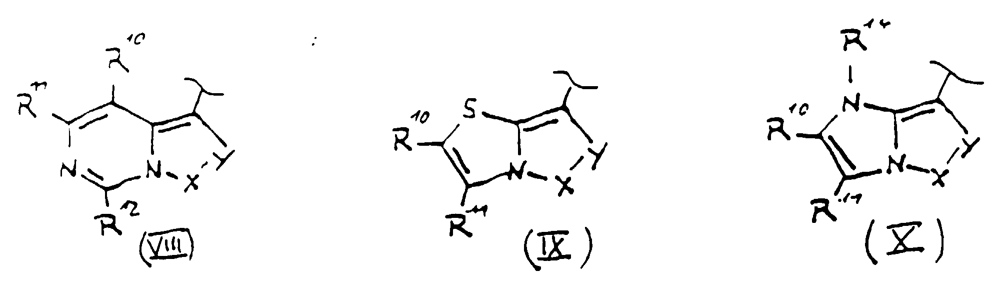 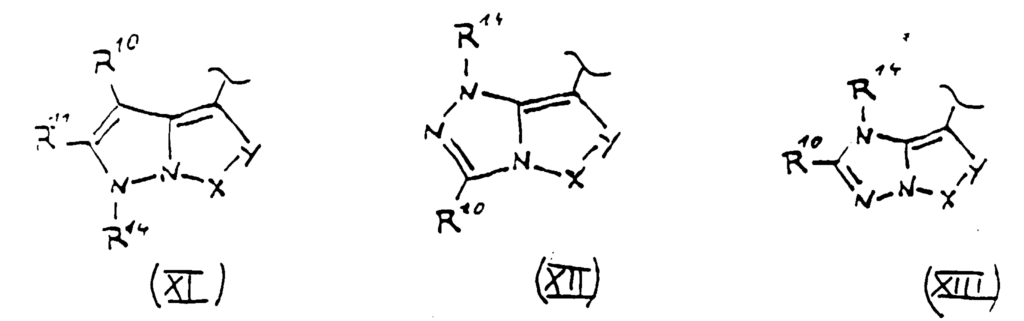 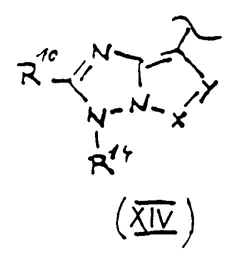 in which R¹⁰, R¹¹, R¹² and R¹³ which are the same or different represent hydrogen, hydroxy, C₁-C₆ alkyl, C₁-C₆ alkoxy, C₁-C₆ alkylthio, C₆-C₁₀ ar-(C₁-C₆) alkyl, C₆-C₁₀ aryl, carboxy, C₁-C₆ alkoxycarbonyl, carboxamido, cyano, amino which is substituted if desired by one or two C₁-C₆ alkyl residues optionally carrying one or several hydroxy, carboxy or/and C₁-C₆ alkoxycarbonyl residues or they represent halogen, wherein two adjacent residues if desired form a C₃-C₄ alkylene group which in turn is substituted if desired by C₆-C₁₀ aryl or is anellated and R¹⁴ represents C₁-C₆ alkyl or C₆-C₁₀ ar-(C₁-C₆)alkyl which is substituted if desired by hydroxy, carboxy, SO₃H, PO₃H₂ or di-(C₁-C₆)alkylphosphinyl.

6. Nitrosoaniline compound of formula I, 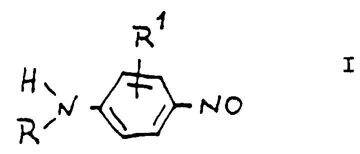 in which
R¹ denotes hydrogen, halogen, hydroxy, C₁-C₆ alkoxy, C₆-C₁₀ aryloxy, C₁-C₆ alkylthio or C₆-C₁₀ arylthio, C₁-C₆ alkyl or C₂-C₅ alkylene substituted if desired by carboxy, PO₃H₂, di(C₁-C₆)alkyl-phosphinyl or SO₃H, amino substituted if desired once or twice by C₁-C₆ alkyl which in turn can be substituted if desired by hydroxy, PO₃H₂, SO₃H or carboxy and
R denotes a residue which forms a colour by electron conjugation with a quinone diimiine system.

7. Nitrosoaniline compound as claimed in claim 6, wherein R represents a residue of the general formula III 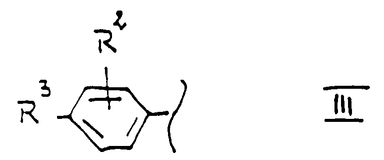 in which
R² denotes hydrogen, hydroxy, halogen, C₁-C₆ alkoxy or C₁-C₆ alkylthio, C₆-C₁₀ aryloxy or C₆-C₁₀ arylthio, C₁-C₆ alkyl or C₂-C₅ alkylene, optionally substituted by carboxy, PO₃H₂ or SO₃H, amino which is substituted if desired once or twice by C₁-C₆ alkyl which in turn can be substituted if desired by hydroxy, PO₃H₂, SO₃H or carboxy, or can form a -CH₂-CH₂-, -CH₂- O-, -CR¹²=CR¹³-, -O-, -S- or -NH-bridge with the residue R¹ in which-NH-can be substituted if desired by C₁-C₆ alkyl and R¹² and R¹³ denote carboxy or C₁-C₆ alkyl which can be substituted if desired by hydroxy, carboxy, PO₃H₂ or SO₃H and
R³ denotes a hydroxy group, C₁-C₆ alkyl, C₁-C₆ alkoxy, C₆-C₁₀ aryl, C₆-C₁₀ aryloxy, C₆-C₁₀ arylthio or C1-C6 alkylthio group wherein if desired the alkyl residue is in turn substituted if desired by a hydroxy group, a C₁-C₆ alkoxy group, an amino group substituted if desired once or several times by C₁-C₆ alkyl, PO₃H₂, SO₃H or CO₂H as such or in a salt form as ammonium, alkali or alkaline earth salts,
or denotes an amino group NR⁴R⁵,
in which R⁴ and R⁵ can be the same or different and denote hydrogen, a C₁-C₆ alkyl group which in turn can be substituted by a hydroxy, C₁-C₆ alkoxy, hydroxy-(C₁-C₆)alkoxy, a poly-(C₁-C₆) alkoxy group substituted if desired by hydroxy, PO₃H₂, SO₃H, COOH as such or in a salt form or by an amino group substituted if desired once or several times by C₁-C₆ alkyl, or
in which R⁴ and R⁵ can represent a C₂-C₅ alkylene residue which if desired is interrupted by oxygen, sulphur or nitrogen, wherein nitrogen is substituted by a C₁-C₆ alkyl, hydroxy-(C₁-C₆)alkyl, hydroxy-(C₁-C₆)alkoxy-(C₁-C₆)alkyl, C₁-C₆ alkoxy-hydroxy-(C₁-C₆)alkyl, C₁-C₆ alkoxycarbonyl-(C₁-C₆)alkyl, dioxanylyl-(C₁-C₆)alkyl or poly-(C₁-C₆)alkoxy-(C₁-C₆)alkyl residue each of which in turn can if desired be substituted in the alkyl moiety by a hydroxy residue, or if R² is in the ortho position in relation to NR⁴R⁵, R⁴ or R⁵ together with R² can also represent a C₂-C₅ alkylene residue.

8. Nitrosoaniline compound as claimed in claim 6, wherein R represents a residue of the general formula IV 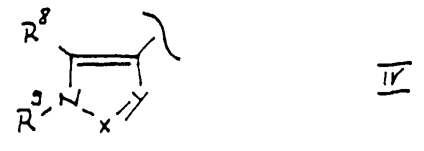 wherein
X-Y represents NR⁶-CO or N=CR⁷ in which
R⁶ represents H, C₁-C₆ alkyl optionally substituted by hydroxy, carboxy, SO₃H, PO₃H₂, di-(C₁-C₆)alkylphosphinyl,
R⁷ represents H, C₁-C₆ alkyl, C₂-C₅ alkenyl, C₁-C₆ alkoxy, C₁-C₆ alkylthio, C₆-C₁₀ aryl, C₆-C₁₀ ar-(C₁-C₆)alkyl, optionally substituted by hydroxy, di-(C₁-C₆)alkylphosphinyl, carboxy, SO₃H, PO₃H₂, a soft of one of these acid residues or/and alkoxycarbonyl, or
amino which if desired is substituted by one or two C₁-C₆ alkyl residues carrying if desired one or several hydroxy, carboxy or/and C₁-C₆ alkoxycarbonyl residues, wherein if amino is substituted by 2 C₁-C₆ alkyl residues these residues can also be linked to form a ring which, apart from the N atom of the amino group, can also be interrupted by oxygen, sulphur or a further nitrogen atom or amino is substituted if desired by one or two acyl groups, C₁-C₆ alkoxy or/and C₆-C₁₀ ar-(C₁-C₆)alkoxycarbonyl groups, H₂N-CO, C₁-C₆ alkyl, C₆-C₁₀ ar-(C₁-C₆)alkyl or/and C₆-C₁₀ arylcarbamoyl groups; or is hydrogen, carboxy, C₁-C₆ alkoxy-carbonyl, carboxamido or halogen and
R⁸ represents C₁-C₆ alkyl, C₁-C₆ thioalkyl or C₆-C₁₀ ar-(C₁-C₆)alkyl, substituted if desired by hydroxy, carboxy, SO₃H or PO₃H₂ or amino which is substituted if desired by one or two C₁-C₆ alkyl groups which in turn can be substituted by hydroxy, carboxy, SO₃H, di-(C₁-C₆)alkylphosphinyl or PO₃H₂, wherein at least R⁷ and/or R⁸ represent an amino group, and
R⁹ represents a C₁-C₆ alkyl or C₆-C₁₀ ar-(C₁-C₆)alkyl group which can be substituted if desired by hydroxy, carboxy, SO₃H or PO₃H₂
or wherein R⁸ and R⁹ together
represent a saturated or unsaturated chain with 3 or 4 members composed of nitrogen atoms or carbon atoms and if desired of one or several nitrogen or sulphur atoms, wherein the carbon atoms are substituted if desired by C₁-C₆ alkyl, C₁-C₆ alkoxy, C₁-C₆ alkylthio, hydroxy, C₆-C₁₀ ar-(C₁-C₆)alkyl, C₁-C₆ aryl, carboxy, carboxamido, C₁-C₆ alkoxycarbonyl, cyano, halogen, amino which is substituted if desired by one or two C₁-C₆ alkyl residues carrying if desired one or several hydroxy, carboxy or/and C₁-C₆ alkoxycarbonyl residues and wherein nitrogen atoms which are not bound via a double bond are substituted by hydrogen, C₁-C₆ alkyl optionally substituted by hydroxy, SO₃H, PO₃H₂, COOH or di-(C₁-C₆)alkylphosphinyl or by C₆-C₁₀ ar-(C₁-C₁₀)alkyl or two adjacent chain substituents optionally form a C₃-C₄ alkylene group which in turn is substituted if desired by C₆-C₁₀ aryl or is anellated, as well as if desired corresponding tautomeric forms and their salts.

9. Agent for the colorimetric determination of NAD(P)H which is composed of an enzyme oxidizing NAD(P)H and of a direct chromogenic electron acceptor, wherein it contains benzyl alcohol dehydrogenase and a nitrosoaniline compound as claimed in one of the claims 6 - 8.

10. Agent for the colorimetric determination of benzyl alcohol dehydrogenase which contains NAD(P)H and a direct chromogenic electron acceptor, wherein the electron acceptor is a nitrosoaniline compound as claimed in one of the claims 6 - 8.

11. Agent for the colorimetric determination of a NAD(P)⁺-dependent dehydrogenase which contains a substrate of the dehydrogenase to be determined, NAD(P)⁺, a NAD(P)H-converting dehydrogenase and a direct chromogenic electron acceptor, wherein the NAD(P)H-converting dehydrogenase is benzyl alcohol dehydrogenase and the electron acceptor is a nitrosoaniline compound as claimed in one of the claims 6 - 8.

12. Agent for the colorimetric determination of an analyte which is a substrate of an enzyme which reacts with concomitant formation of NAD(P)H containing an analyte-specific, NAD(P)-dependent dehydrogenase, NAD(P), a NAD(P)H-oxidizing enzyme and a direct chromogenic electron acceptor, wherein it contains benzyl alcohol dehydrogenase as the NAD(P)H-oxidizing enzyme and a nitrosoaniline compound as claimed in one of the claims 6 - 8 as the direct chromogenic electron acceptor.

13. Process for the production of the nitrosoaniline compound of formula I as claimed in claim 6, wherein a p-nitrosophenyl ether of formula XVII is reacted with an amino compound of formula XVIII R-NH₂.

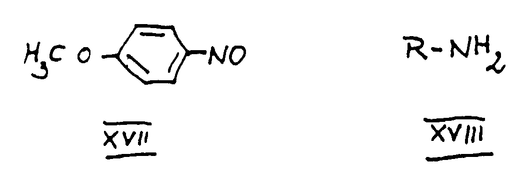

14. Use of nitrosoaniline compounds as claimed in claims 6 - 8 for the colorimetric enzymatic determination of an analyte.

## Revendications

1. Procédé de détermination colorimétrique de NAD(P)H ou d'un analyte qui est un substrat d'une enzyme réagissant en formant du NAD(P)H, par oxydation à l'aide d'une enzyme oxydant NAD(P)H, en présence d'un accepteur d'électrons chromogène direct, et détermination de l'accepteur d'électrons réduit comme mesure de la quantité de NAD(P)H, caractérisé en ce que le NAD(P)H est mis à réagir, en tant qu'accepteur d'électrons. avec la déshydrogénase de l'alcool benzylique, en présence d'un composé nitrosoaniline de formule I 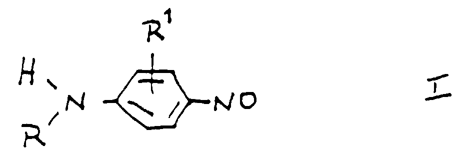 dans laquelle
R¹ représente un atome d'hydrogène, d'halogène, un groupe hydroxy, alcoxy en C₁-C₆, aryloxy en C₆-C₁₀, alkylthio en C₁-C₆ ou arylthio en C₆-C₁₀, alkyle en C₁-C₆ ou alkylène en C₂-C₅, éventuellement substitué par un groupe carboxy, PO₃H₂, di(alkyle en C₁-C₆)phosphinyle ou SO₃H, amino éventuellement substitué une ou deux fois par un groupe alkyle en C₁-C₆ qui, à son tour, peut être éventuellement substitué par un groupe hydroxy, PO₃H₂, di(alkyle en C₁-C₆)phosphinyle, SO₃H ou carboxy, et
R représente un reste chromogène par conjugaison d'électrons avec un système quinonediimine,
le composé nitrosoaniline étant alors réduit par voie enzymatique en une quinonediimine colorée et la couleur de la quinonodiimine est déterminée comme mesure de la quantité de NAD(P)H.

2. Procédé selon la revendication 1, caractérisé en ce que, en tant que reste chromogène, on utilise un reste de formule III 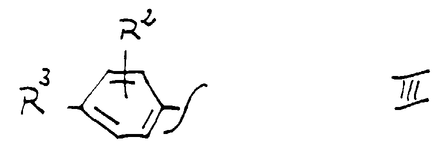 dans laquelle
R² représente un atome d'hydrogène, un groupe hydroxy, un atome d'halogène, un groupe alcoxy en C₁-C₆, alkylthio en C₁-C₆, aryloxy en ou arylthio en C₆-C₁₀, alkyle en C₁-C₆ ou alkylène en C₂-C₅, éventuellement substitué par un groupe carboxy, PO₃H₂ ou SO₃H, amino éventuellement substitué une ou deux fois par un groupe alkyle en C₁-C₆ qui, à son tour, peut être éventuellement substitué par un groupe hydroxy, PO₃H₂, SO₃H ou carboxy, ou peut former, avec le reste R¹, un pont -CH₂-CH₂-, -CH₂-O-, -CR¹²=CR¹³-, -O-, -S- ou -NH-,-NH- pouvant être éventuellement substitué par un reste alkyle en C₁-C₆ et R¹² et R¹³ représente un groupe carboxy ou alkyle en C₁-C₆ qui, à son tour, peut être éventuellement substitué par un groupe hydroxy, carboxy, PO₃H₂ ou SO₃H.
R³ représente un groupe hydroxy, alkyle en C₁-C₆, alcoxy en C₁-C₆, aryle en C₆-C₁₀, aryloxy en C₆-C₁₀, arylthio en C₆-C₁₀ ou alkylthio en C₁-C₆; le reste alkyle pouvant être éventuellement substitué à son tour par un groupe hydroxy, alcoxy en C₁-C₆, amino éventuellement substitué une ou plusieurs fois par un groupe alkyle en C₁-C₆, PO₃H₂, SO₃H ou CO₂H, tel quel ou sous la forme d'un sel tel qu'un sel d'ammonium, un sel de métal alcalin ou alcalinoterreux,
ou représente un groupe amino NR⁴R⁵,
où R⁴ et R⁵ peuvent être identiques ou différents et représentent un atome d'hydrogène, un groupe alkyle en C₁-C₆ qui, à son tour, peut être substitué par un groupe hydroxy, alcoxy en C₁-C₆, hydroxy(alcoxy en C₁-C₆), un groupe poly(alcoxy en C₁-C₆) éventuellement substitué par un groupe hydroxy, PO₃H₂, SO₃H, COOH, tel quel ou sous la forme d'un sel, ou un groupe amino éventuellement substitué une ou plusieurs fois par un groupe alkyle en C₁-C₆,
ou
où R⁴ et R⁵ peuvent représenter un reste alkylène en C₂-C₅, qui peut être interrompu éventuellement par un atome d'oxygène, de soufre ou d'azote, l'atome d'azote étant substitué par un groupe alkyle en C₁-C₆, hydroxy(alkyle en C₁-C₆), hydroxy(alcoxy en C₁-C₆)(alkyle en C₁-C₆), (alcoxy en C₁-C₆)-hydroxy(alkyle en C₁-C₆), (alcoxy en C₁-C₆)carbonyl(alkyle en C₁-C₆), dioxyanylyl(alkyle en C₁-C₆) ou poly(alcoxy en C₁-C₆)-(alkyle en C₁-C₆), qui peuvent être chacun éventuellement substitués à leur tour par un reste hydroxy sur la partie alkyle, ou lorsque R² est en position ortho par rapport à NR⁴R⁵, R⁴ et R⁵ peuvent également représenter, conjointement avec R², un reste alkylène en C₂-C₅.

3. Procédé selon la revendication 2, caractérisé en ce que R représente un groupe N,N-dihydroxyéthylaniline, N,N-diméthylaniline ou -N,N-diéthylaniline.

4. Procédé selon la revendication 1, caractérisé en ce que, en tant que reste chromogène, on utilise un reste de formule générale IV 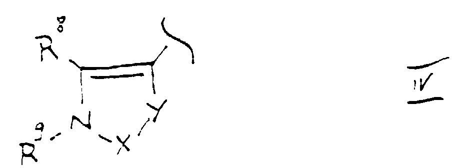 dans laquelle
X-Y représente NR⁶CO ou N=CR⁷, où
R⁶ représente un atome de H, un groupe alkyle en C₁-C₆ éventuellement substitué par un groupe hydroxy, carboxy, SO₃H, PO₃H₂, di(alkyle en C₁-C₆)phosphinyle,
R⁷ représente un atome de H, un groupe alkyle en C₁-C₆, alcényle en C₂-C₅, alcoxy en C₁-C₆, alkylthio en C₁-C₆, aryle en C₆-C₁₀, (aryle en C₆-C₁₀)(alkyle en C₁-C₆), éventuellement substitué par un groupe hydroxy, di(alkyle en C₁-C₆)phosphinyle, carboxy, SO₃H, PO₃H₂, un sel de ces restes d'acides et/ou un groupe (alcoxy en C₁-C₆)carbonyle, ou
un groupe amino, qui peut être éventuellement substitué par un ou deux restes alkyle en C₁-C₆, portant éventuellement un ou plusieurs groupes hydroxy, carboxy et/ou alcoxy en C₁-C₆, où, lorsque le groupe amino est substitué par 2 restes alkyle en C₁-C₆, ces restes peuvent également se lier pour former un cycle qui, en plus de l'atome de N du groupe amino, peut être éventuellement interrompu par un atome d'oxygène, un atome de soufre ou un autre atome d'azote, ou le groupe amino est substitué éventuellement par un ou deux groupes acyle, alcoxy en C₁-C₆ et/ou aryle(alcoxy en C₁-C₆)carbonyle, H₂NCO, alkyle en C₁-C₆, (aryle en C₆-C₁₀)(alkyle en C₁-C₆) et/ou (aryle en C₆-C₁₀)carbamoyle ; ou un atome d'hydrogène, un groupe carboxy, (alcoxy en C₁-C₆)carbonyle, carboxamido ou halogéno, et
R⁸ représente un groupe alkyle en C₁-C₆, thio(alkyle en C₁-C₆) ou (aryle en C₆-C₁₀)(alkyle en C₁-C₆), éventuellement substitué par un groupe hydroxy, carboxy, SO₃H ou PO₃H₂ ou amino, qui est éventuellement substitué par un ou deux groupes alkyle en C₁-C₆ qui, à leur tour, peuvent être substitués par un groupe hydroxy, carboxy SO₃H, di(alkyle en C₁-C₆)phosphinyle ou PO₃H₂, au moins R⁷ et/ou R⁸ représentant un groupe amino, et
R⁹ représente un groupe alkyle en C₁-C₆ ou (aryle en C₆-C₁₀)-(alkyle en C₁-C₆), qui peut être éventuellement substitué par un groupe hydroxy, carboxy, SO₃H ou PO₃H₂,
ou R⁸ et R⁹ peuvent former ensemble une chaîne saturée ou insaturée, comprenant 3 ou 4 maillons, formée d'atomes d'azote ou d'atomes de carbone et éventuellement d'un ou plusieurs atomes d'azote ou de soufre, les atomes de carbone étant éventuellement substitués par un groupe alkyle en C₁-C₆, alcoxy en C₁-C₆, alkylthio en C₁-C₆, hydroxy, (aryle en C₆-C₁₀)(alkyle en C₁-C₆), aryle en C₆-C₁₀, carboxy, carboxamido, (alcoxy en C₁-C₆)carbonyle, cyano, halogéno, amino éventuellement substitué une ou deux fois par un groupe alkyle en C₁-C₆ qui, à son tour, peut être éventuellement substitué par un groupe hydroxy, carboxy et/ou (alcoxy en C₁-C₆)carbonyle), et les atomes d'azote qui ne sont pas liés par l'intermédiaire de doubles liaisons, sont substitués par un atome d'hydrogène, un groupe alkyle en C₁-C₆ qui, à son tour, peut être éventuellement substitué par un groupe hydroxy, SO₃H, PO₃H₂, COOH ou di(alkyle en C₁-C₆)phosphinyle, ou par un groupe (aryle en C₆-C₁₀)(alkyle en C₁-C₆), ou deux substituants de chaîne contigüs forment éventuellement un groupe alkylène en C₃-C₄ qui, à son tour, peut être
éventuellement substitué par. ou annellé à, un groupe aryle en C₆-C₁₀,
ainsi qu'éventuellement les formes tautomères correspondantes et leurs sels.

5. Procédé selon la revendication 4, caractérisé en ce que R représente un reste répondant aux formules de V à XIV 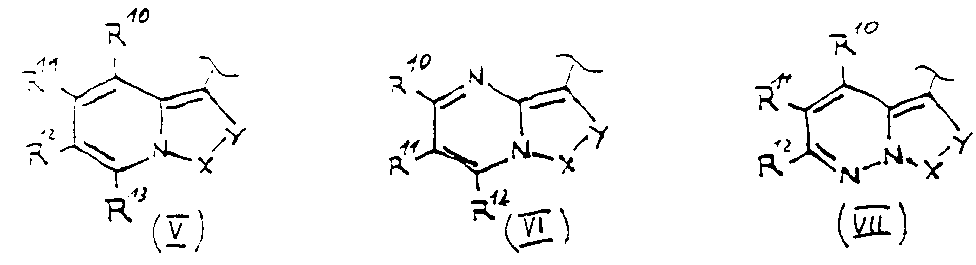 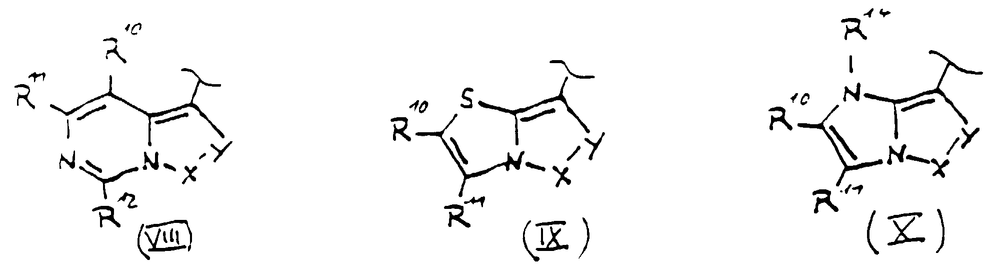 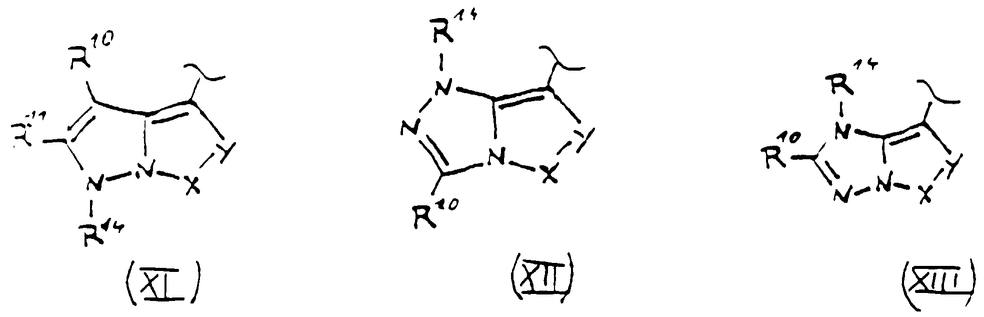 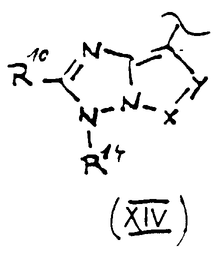 dans lesquelles R¹⁰, R¹¹, R¹² et R¹³, qui peuvent être identiques ou différents, représentent un atome d'hydrogène, un groupe hydroxy, un groupe alkyle en C₁-C₆, alcoxy en C₁-C₆, alkylthio en C₁-C₆, (aryle en C₆-C₁₀(alhyle en C₁-C₆), aryle en C₆-C₁₀, carboxy, (alcoxy en C₁-C₆)carbonyle, carboxamido, cyano, amino, qui peut être éventuellement substitué par un ou deux restes alkyle en C₁-C₆ portant un ou plusieurs groupes hydroxy, carboxy et/ou (alcoxy en C₁-C₆)carbonyle, ou un groupe halogéno, deux restes contigüs formant éventuellement un groupe alkylène en C₃-C₄, qui à son tour peut être éventuellement substitué par, ou annellé à, un groupe aryle en C₆-C₁₀, et R¹⁴ représente un groupe alkyle en C₁-C₆ ou (aryle en C₆-C₁₀)(alkyle en C₁-C₆), qui est éventuellement substitué par un groupe hydroxy, carboxy, SO₃H, PO₃H₂ ou di(alkyle en C₁-C₆)phosphinyle.

6. Composé nitrosoaniline de formule 1 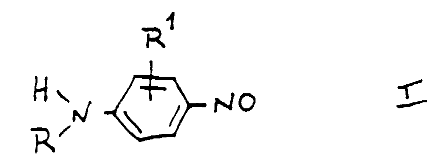 dans laquelle
R¹ représente un atome d'hydrogène, d'halogène, un groupe hydroxy, alcoxy en C₁-C₆, aryloxy en C₆-C₁₀, alkylthio en C₁-C₆ ou arylthio en C₆-C₁₀, alkyle en C₁-C₆ ou alkylène en C₂-C₅, éventuellement substitué par un groupe carboxy, PO₃H₂, di(alkyle en C₁-C₆)phosphinyle ou SO₃H, amino éventuellement substitué une ou deux fois par un groupe alkyle en C₁-C₆ qui, à son tour, peut être éventuellement substitué par un groupe hydroxy, PO₃H₂, SO₃H ou carboxy, et
R représente un reste chromogène par conjugaison d'électrons avec un système quinonediimine.

7. Composé nitrosoaniline selon la revendication 6, caractérisé en ce que R représente un reste de formule générale III 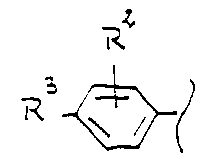 dans laquelle
R² représente un atome d'hydrogène, un groupe hydroxy, un atome d'halogène, un groupe alcoxy en C₁-C₆, alkylthio en C₁-C₆, aryloxy ou arylthio en C₆-C₁₀, alkyle en C₁-C₆ ou alkylène en C₂-C₅, éventuellement substitué par un groupe carboxy, PO₃H₂ ou SO₃H, amino éventuellement substitué une ou deux fois par un groupe alkyle en C₁-C₆ qui, à son tour, peut être éventuellement substitué par un groupe hydroxy, PO₃H₂, SO₃H ou carboxy, ou peut former, avec le reste R¹, un pont -CH₂-CH₂-, -CH₂-O-, -CR¹²=CR¹³-, -O-, -S- ou -NH-, -NH- pouvant être éventuellement substitué par un reste alkyle en C₁-C₆ et R¹² et R¹³ représente un groupe carboxy ou alkyle en C₁-C₆ qui, à son tour, peut être éventuellement substitué par un groupe hydroxy, carboxy, PO₃H₂ ou SO₃H,
R³ représente un groupe hydroxy, alkyle en C₁-C₆, alcoxy en C₁-C₆, aryle en C₆-C₁₀, aryloxy en C₆-C₁₀, arylthio en C₆-C₁₀ ou alkylthio en C₁-C₆, le reste alkyle pouvant être éventuellement substitué à son tour par un groupe hydroxy, alcoxy en C₁-C₆, amino éventuellement substitué une ou plusieurs fois par un groupe alkyle en C₁-C₆, PO₃H₂, SO₃H ou CO₂H, tel quel ou sous la forme d'un sel tel qu'un sel d'ammonium, un sel de métal alcalin ou alcalinoterreux,
ou représente un groupe amino NR⁴R⁵,
où R⁴ et R⁵ peuvent être identiques ou différents et représentent un atome d'hydrogène, un groupe alkyle en C₁-C₆ qui, à son tour, peut être substitué par un groupe hydroxy, alcoxy en C₁-C₆, hydroxy(alcoxy en C₁-C₆), un groupe poly(alcoxy en C₁-C₆) éventuellement substitué par un groupe hydroxy, PO₃H₂, SO₃H, COOH, tel quel ou sous la forme d'un sel. ou un groupe amino éventuellement substitué une ou plusieurs fois par un groupe alkyle en C₁-C₆,
ou
où R⁴ et R⁵ peuvent représenter un reste alkylène en C₂-C₅, qui peut être interrompu éventuellement par un atome d'oxygène, de soufre ou d'azote, l'atome d'azote étant substitué par un groupe alkyle en C₁-C₆, hydroxy(alkyle en C₁-C₆), hydroxy(alcoxy en C₁-C₆)(alkyle en C₁-C₆), (alcoxy en C₁-C₆)-hydroxy(alkyle en C₁-C₆), (alcoxy en C₁-C₆)carbonyl(alkyle en C₁-C₆), dioxyanylyl(alkyle en C₁-C₆) ou poly(alcoxy en C₁-C₆)-(alkyle en C₁-C₆), qui peuvent être chacun éventuellement substitués à leur tour par un reste hydroxy sur la partie alkyle, ou lorsque R² est en position ortho par rapport à NR⁴R⁵, R⁴ et R⁵ peuvent également représenter, conjointement avec R², un reste alkylène en C₂-C₅.

8. Composé nitrosoaniline selon la revendication 6, caractérisé en ce que R représente un reste de formule générale IV 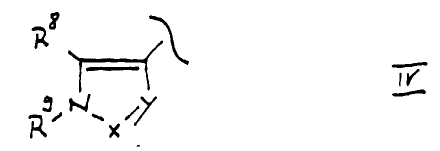 dans laquelle
X-Y représente NR⁶CO ou N=CR⁷, où
R⁶ représente un atome de H, un groupe alkyle en C₁-C₆ éventuellement substitué par un groupe hydroxy, carboxy, SO₃H, PO₃H₂, di(alkyle en C₁-C₆)phosphinyle,
R⁷ représente un atome de H, un groupe alkyle en C₁-C₆, alcényle en C₂-C₅, alcoxy en C₁-C₆, alkylthio en C₁-C₆, aryle en C₆-C₁₀, (aryle en C₆-C₁₀)(alkyle en C₁-C₆), éventuellement substitué par un groupe hydroxy, di(alkyle en C₁-C₆)phosphinyle, carboxy, SO₃H, PO₃H₂, un sel de ces restes d'acides et/ou un groupe (alcoxy en C₁-C₆)carbonyle, ou
un groupe amino, qui peut être éventuellement substitué par un ou deux restes alkyle en C₁-C₆, portant éventuellement un ou plusieurs groupes hydroxy, carboxy et/ou alcoxy en C₁-C₆, où, lorsque le groupe amino est substitué par 2 restes alkyle en C₁-C₆, ces restes peuvent également se lier pour former un cycle qui, en plus de l'atome de N du groupe amino, peut être éventuellement interrompu par un atome d'oxygène, un atome de soufre ou un autre atome d'azote. ou le groupe amino est substitué éventuellement par un ou deux groupes acyle, alcoxy en C₁-C₆ et/ou (aryle en C₆-C₁₀)(alcoxy en C₁-C₆)-carbonyle, H₂NCO, alkyle en C₁-C₆, (aryle en C₆-C₁₀)(alkyle en C₁-C₆) et/ou (aryle en C₆-C₁₀)carbamoyle; ou un atome d'hydrogène, un groupe carboxy, (alcoxy en C₁-C₆)carbonyle, carboxamido ou halogéno, et
R⁸ représente un groupe alkyle en C₁-C₆, thio(alkyle en C₁-C₆) ou (aryle en C₆-C₁₀)(alkyle en C₁-C₆), éventuellement substitué par un groupe hydroxy, carboxy, SO₃H ou PO₃H₂ ou amino, qui est éventuellement substitué par un ou deux groupes alkyle en C₁-C₆ qui, à leur tour, peuvent être substitués par un groupe hydroxy, carboxy, SO₃H, di(alkyle en C₁-C₆)phosphinyle ou PO₃H₂, au moins R⁷ et/ou R⁸ représentant un groupe amino, et
R⁹ représente un groupe alkyle en C₁-C₆ ou (aryle en C₆-C₁₀)-(alkyle en C₁-C₆), qui peut être éventuellement substitué par un groupe hydroxy, carboxy, SO₃H ou PO₃H₂,
ou R⁸ et R⁹ peuvent former ensemble
une chaîne saturée ou insaturée, comprenant 3 ou 4 maillons, formée d'atomes d'azote ou d'atomes de carbone et éventuellement d'un ou plusieurs atomes d'azote ou de soufre, les atomes de carbone étant éventuellement substitués par un groupe alkyle en C₁-C₆, alcoxy en C₁-C₆, alkylthio en C₁-C₆, hydroxy, (aryle en C₆-C₁₀)(alkyle en C₁-C₆), aryle en C₆-C₁₀, carboxy, carboxamido, (alcoxy en C₁-C₆)carbonyle) cyano, halogéno, amino éventuellement substitué une ou deux fois par un groupe alkyle en C₁-C₆ qui, à son tour, peut être éventuellement substitué par un groupe hydroxy, carboxy et/ou (alcoxy en C₁-C₆)carbonyle) et les atomes d'azote qui ne sont pas liés par l'intermédiaire de doubles liaisons, sont substitués par un atome d'hydrogène, un groupe alkyle en C₁-C₆ qui, à son tour, peut être éventuellement substitué par un groupe hydroxy, SO₃H, PO₃H₂, COOH ou di(alkyle en C₁-C₆)phosphinyle, ou par un groupe (aryle en C₆-C₁₀)(alkyle en C₁-C₆), ou deux substituants de chaîne contigüs forment éventuellement un groupe alkylène en C₃-C₄ qui, à son tour, peut être éventuellement substitué par, ou annellé à, un groupe aryle en C₆-C₁₀,
ainsi qu'éventuellement les formes tautomères correspondantes et leurs sels.

9. Agent destiné à la détermination colorimétrique de NAD(P)H, constitué par une enzyme oxydant NAD(P)H et un accepteur d'électrons chromogène direct, caractérise en ce qu'il contient la déshydrogénase de l'alcool benzylique et un composé nitrosoaniline selon l'une quelconque des revendications 6-8.

10. Agent destiné à la détermination colorimétrique de la déshydrogénase de l'alcool benzylique. contenant du NAD(P)H et un accepteur d'électrons chromogène direct, caractérisé en ce que l'accepteur d'électrons est un composé nitrosoaniline selon l'une quelconque des revendications 6-8.

11. Agent destiné à la détermination colorimétrique d'une déshydrogénase dépendant de NAD(P)⁺, contenant un substrat de la déshydrogénase à déterminer, NAD(P)⁺, une déshydrogénase réagissant avec NAD(P)H et un accepteur d'électrons chromogène direct, caractérisé en ce que la déshydrogénase réagissant avec NAD(P)H est la déshydrogénase de l'alcool benzylique et l'accepteur d'électrons est un composé nitrosoaniline selon l'une quelconque des revendications 6-8.

12. Agent destiné à la détermination colorimétrique d'un analyte qui est un substrat d'une enzyme réagissant en formant NAD(P)H, contenant une déshydrogénase dépendant de NAD(P)H, spécifique à l'analyte, NAD(P), une enzyme oxydant NAD(P)H et un accepteur d'électrons chromogène direct, caractérisé en ce que, en tant d'enzyme oxydant NAD(P)H, il contient la déshydrogénase de l'alcool benzylique et en tant qu'accepteur d'électrons chromogène direct, il contient un composé nitrosoaniline selon l'une quelconque des revendications 6-8.

13. Procédé de préparation du composé nitrosoaniline de formule I selon la revendication 6, caractérisé en ce que l'on fait réagir un p-nitrosophényléther de formule XVII avec un composé amino de formule XVIII RNH₂.

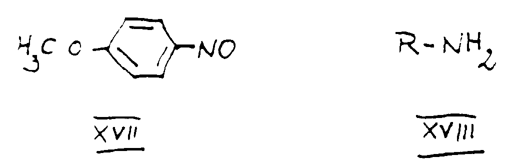

14. Utilisation de composés nitrosoaniline selon l'une quelconque des revendications 6 à 8, pour la détermination enzymatique colorimétrique d'un analyte.
